# EUROPEAN PATENT SPECIFICATION

(11) **EP 4 039 296 B1**
(45) Date of publication and mention of the grant of the patent: **16.07.2025**
(21) Application number: 20870726.5
(22) Date of filing: 30.09.2020
(51) Int. Cl.: A61M 11/00, A61M 11/08, A61M 15/00, A61M 15/08, A61M 11/02

(54) **NASAL SPRAY/SPOUT NOZZLE AND NASAL REST ARTICLE FOR NASAL ADMINISTRATION**
NASENSPRAY-/AUSGUSSDÜSE UND NASENSTÜTZARTIKEL ZUR NASALEN VERABREICHUNG
BUSE DE PULVÉRISATION NASALE/DE SORTIE ET ARTICLE DE REPOS NASAL POUR ADMINISTRATION NASALE

(30) Priority: 01.10.2019 JP 2019181591
(43) Date of publication of application: 10.08.2022
(73) Proprietor: Toko Yakuhin Kogyo Co., Ltd., Osaka-shi, Osaka 530-0022 (JP)
(72) Inventor: KAMISHITA, Taizou, Osaka-shi, Osaka 530-0022 (JP); MIYAZAKI, Takashi, Osaka-shi, Osaka 530-0022 (JP)
(74) Representative: Hoffmann Eitle
(86) International application number: PCT/JP2020/037930
(87) International publication number: WO 2021/066195

(56) References cited:
- WO-A1-2009/057572
- WO-A1-2017/021878
- WO-A1-2018/025139
- FR-A1- 2 739 294
- JP-A- 2002 510 225
- JP-A- 2012 135 535
- JP-A- 2018 187 412
- JP-U- 3 112 243
- JP-U- H03 129 153
- JP-U- H03 129 153
- US-A1- 2002 174 865
- US-B1- 6 427 680

## Description

### TECHNICAL FIELD

The present invention relates to a nasal spray/spout nozzle for administering a medicament into a nasal cavity, and also relates to a nasal rest article for such administration of the medicament.

### BACKGROUND ART

Conventionally, an administration of a medicament has been widely performed in such a way that a spray/spout device is charged with a liquid agent, followed by spraying or spouting of the liquid agent onto a mucosa or skin of a body lumen/cavity or body surface by means of the device. In particular, the medicament for an intranasal administration has been widely available.

The merits on the medicament for spray/ spout administration with respect to the mucosa, especially the merits of the medicament for intranasal administration include that (1) a fast-acting effect of the medicament is expected due to a rapid absorption of the medicament; (2) it is possible to avoid a decomposition of the medicament, the decomposition being attributed to the first-pass in liver; (3) it is further possible to avoid another decomposition of the medicament, the decomposition being attributed to gastric acid or gastrointestinal enzyme in gastrointestinal tract; (4) it is possible to reduce a dose of the medicament because of its high bioavailability; (5) a non-invasive administration can be realized as compared with a needle injection; (6) a patient can treat himself or herself; (7) the medicament can more directly act on the blood circulation or central nervous system; and (8) a nasal mucosa has a specific immunological defense mechanism based on a non-specific immunological defense mechanism, and thereby bringing out a suitable availability of nasal vaccines, and so on.
US 6 427 680 B1 relates to a nasal applicator and to a dispenser device including such an applicator for dispensing a fluid or a powder substance into the nose.FR 2 739 294 A1 relates to a safety nasal tip intended to introduce by jet or spray, a medicinal solution or suspension into the nose of a patient.
WO 2018/025139 A1 relates to a dispenser for dispensing nebulized liquid solutions.
WO 2017/021878 A1 relates to devices for nasal irrigation, and in particular a safety device for nasal irrigation provided with a pressure and flow rate limiting device.
US 2002/174865 A1 relates to a nasal spray system characterized by features of construction and arrangement providing ease of insertion of the device in the nostril in a manner to allow easy inhalation and a particular rib configuration which creates turbulence to atomize the nasal spray liquid.

### SUMMARY OF THE INVENTION

### PROBLEMS TO BE SOLVED BY THE INVENTION

A medicament for intranasal administration is mainly expected to give a local effect as a rhinitis treatment or the like by being supplied onto a respiratory region (e.g., nasal turbinate). These days, the medicament for intranasal administration has been more widely used. For example, it has been used for purpose of a medicament administration with respect to a nasopharynx region-associated lymphatic tissues having a higher immune response. It also has been used for purpose of the medicament administration with respect to an olfactory region (e.g., olfactory mucosa), and thereby delivering the medicament to a nerve center.

A nasal spray/spout device comprises a container for storing the medicament, a spray/spout nozzle, and a mechanism part (e.g., an actuator) for spraying/spouting a metered dose of the medicament from the container through the spray/spout nozzle. Examples of the nasal spray/spout device include a syringe-type spray/spout device, an airless-type spray/spout device and the like, in addition to a commonly-used conventional nasal spray device.

As the nasal spray/spout device, there is disclosed a syringe-type spray/spout device using a nasal spray/spout nozzle with an improved uniformity of spray/spout density by optimizing a configuration/structure thereof (see JP 6322844 B2), for example. There is also disclosed an upper exhaust airless-type spray/spout device capable of desirably adjusting an administration angle within a desired range (see JP 5185109 B2).

The inventor has found that there is still room for further improving the spray/spout device in terms of a medicament delivery with respect to an intended intranasal region. More specifically, it has been found by the inventor that a medicament spray/spout by use of the nasal spray/spout nozzle can still have room for further improvement in a medicament delivery performance of the nozzle with respect to a target site located in a nasal cavity.

Under these circumstances, the present invention has been created. That is, a main object of the present invention is to provide a nasal spray/spout nozzle capable of further improving the medicament delivery onto the target site located in the nasal cavity.

### MEANS FOR SOLVING THE PROBLEMS

The inventor of this application has addressed the matters described above, from a novel standpoint rather than a continual standpoint of the conventional art. As a result, the inventor of this application has created a nasal spray/spout nozzle that is capable of attaining the main object described above.

According to the present invention, there is provided a nasal spray/spout nozzle for nasal administration according to independent claim 1. Preferred optional features are recited in the dependent claims.

### EFFECT OF THE INVENTION

The nasal spray/spout nozzle according to the present invention can improve the medicament delivery with respect to the target site located in the nasal cavity.

It should, however, be noted that some effects described herein are merely examples, and thus the present invention is not necessarily limited thereto, and thereby additional effect(s) may also be provided.

### BRIEF DESCRIPTION OF THE DRAWINGS

Figs. 1A to 1C are schematic lateral plan views illustrating various embodiments of a nasal spray/spout nozzle according to the present disclosure.
Figs. 2A to 2D are schematic top plan views illustrating various embodiments of a nose rest in a nasal spray/spout nozzle according to the present disclosure.
Figs. 3A to 3D are schematic top plan views illustrating various embodiments of a nose rest in a nasal spray/spout nozzle according to the present disclosure.
Figs. 4A to 4C are schematic views illustrating a nasal spray/spout nozzle according to one embodiment of the present disclosure (Fig. 4A: perspective view, Fig. 4B: lateral plan view, and Fig. 4C: top plan view).
Figs. 5A to 5C are schematic views illustrating a nasal spray/spout nozzle according to one embodiment of the present disclosure (Fig. 5A: perspective view, Fig. 5B: lateral plan view, and Fig. 5C: top plan view).
Figs. 6A to 6C are schematic views illustrating a nasal spray/spout nozzle according to one embodiment of the present disclosure (Fig. 6A: perspective view, Fig. 6B: lateral plan view, and Fig. 6C: top plan view).
Figs. 7A to 7C are schematic views illustrating a nasal spray/spout nozzle according to one embodiment of the present disclosure (Fig. 7A: perspective view, Fig. 7B: lateral plan view, and Fig. 7C: top plan view).
Figs. 8A to 8C are schematic views illustrating a nasal spray/spout nozzle according to one embodiment of the present disclosure (Fig. 8A: perspective view, Fig. 8B: lateral plan view, and Fig. 8C: top plan view).
Figs. 9A to 9C are schematic views illustrating a nasal spray/spout nozzle according to one embodiment of the present disclosure (Fig. 9A: perspective view, Fig. 9B: lateral plan view, and Fig. 9C: top plan view).
Figs. 10A to 10C are schematic views illustrating a nasal spray/spout nozzle according to one embodiment of the present disclosure (Fig. 10A: perspective view, Fig. 10B: lateral plan view, and Fig. 10C: top plan view).
Fig. 11 is a schematic view illustrating a nasal spray/spout nozzle according to one embodiment of the present disclosure.
Figs. 12A to 12C are schematic views illustrating a nasal spray/spout nozzle according to one embodiment of the present disclosure (Fig. 12A: perspective view, Fig. 12B: lateral plan view, and Fig. 12C: top plan view).
Figs. 13A to 13C are schematic views illustrating a nasal spray/spout nozzle according to one embodiment of the present disclosure (Fig. 13A: perspective view, Fig. 13B: lateral plan view, and Fig. 13C: top plan view).
Fig. 14 is a schematic view for explaining that a target region for which a spray/spout is performed by the nasal spray/spout nozzle can be desirably set due to a relationship between a "thin or thick portion" of a nose rest and a "contact region of the thin or thick portion of the nose rest with a nose region located around an external naris".
Figs. 15A to 15C are schematic lateral plan views illustrating nasal spray/spout nozzles according to one embodiment of the present disclosure.
Figs. 16A to 16C are schematic views illustrating a nasal spray/spout nozzle according to one embodiment of the present disclosure. (Fig. 16A: perspective view, Fig. 16B: lateral plan view, and Fig. 16C: top plan view).
Fig. 17 is a schematic view illustrating a nasal spray/spout nozzle according to one embodiment of the present disclosure.
Figs. 18A to 18D are top views of nasal spray/spout nozzles with each of nose rests eccentrically provided therein (Fig. 18A shows the nose rest having a circular shape in a plan view; Fig. 18B shows the nose rest having approximately a trapezoidal shape in a plan view; Fig. 18C shows the nose rest having approximately a gourd-like shape in a plan view; and Fig. 18D shows the nose rest having approximately a pea-like shape in a plan view).
Figs. 19A to 19D are top views of nasal spray/spout nozzles with an outer contour of each tip portion having a non-surround configuration (Fig. 19A shows a nose rest having a circular shape in a plan view; Fig. 19B shows a nose rest having approximately a trapezoidal shape in a plan view; Fig. 19C shows a nose rest having approximately a gourd-like shape in a plan view; and Fig. 19D shows a nose rest having approximately a pea-like shape in a plan view).
Figs. 20A to 20C are top plan views of nasal spray/spout nozzles, each illustrating an outer contour of a nose rest is in a form of arc (Fig. 20A shows the nose rest having an arc shape as a whole while having an elliptical shape in a plan view; Fig. 20B shows the nose rest having an arc shape in a part while having approximately a gourd-like shape in a plan view; and Fig. 20C shows the nose rest having an arc shape in a part while having approximately a pea-like shape in a plan view).
Figs. 21A and 21B are top plan views of nasal spray/spout nozzles in combination of an "embodiment regarding the eccentrically provided nose rest" with an "embodiment regarding the arc-formed outer contour of nose rest" (Fig. 21A shows the nose rest having approximately a gourd-like shape in a plan view; and Fig. 21B shows the nose rest having approximately a pea-like shape in a plan view).
Figs. 22A and 22B are schematic perspective views, each illustrating a use mode of the nozzle with the eccentrically provided nose rest (Fig. 22A shows that a wider portion of the nose rest is oriented such that it becomes an upper side of the nozzle; and Fig. 22B shows that the wider portion of the nose rest is oriented such that it becomes a lower side of the nozzle).
Figs. 23A to 23C are schematic views illustrating a nose rest with its dome form (Fig. 23A: top plan view, Fig. 23B: lateral plan view as viewed in a direction "A", and Fig. 23C: lateral plan view as viewed in a direction "B").
Fig. 24 is a schematic view illustrating a nasal spray/spout nozzle according to one embodiment of the present invention.
Fig. 25 is a schematic perspective view of a nose rest to be used in an eccentric form according to one embodiment of the present invention.
Figs. 26A and 26B are schematic views of a nose rest according to one embodiment of the present disclosure (Fig. 26A: top plan view, and Fig. 26B: sectional view taken along line I-I).
Figs. 27A to 27G are schematic top plan views illustrating various embodiments of a finger rest according to the present disclosure.
Fig. 28 includes schematic sectional views of an upper exhaust airless-type spray/spout device in combination with a nasal spray/spout nozzle according to the present disclosure, in which a right half of the main drawing is shown as a sectional view so as to clarify an interior structure of the device.
Fig. 29 is an enlarged sectional view of a main part of the upper exhaust airless-type spray/spout device shown in Fig. 28.
Figs. 30A to 30F are schematic views for explaining that spray and/or spout can be suitably performed with any suitable administration angle.
Fig. 31 is a sectional view illustrating a schematic structure of a syringe-type spray/spout device in combination with a nasal spray/spout nozzle according to the present disclosure.
Figs. 32A and 32B are exploded perspective views illustrating a schematic configuration of a nasal spray/spout nozzle according to one embodiment of the present disclosure, especially showing the nozzle at a point in time before and after a filling rod is disposed within a body of the nozzle.
Fig. 33A is a cross-sectional view of the nasal spray/spout nozzle of Fig. 32B as viewed in a vertical plane, and Figs. 33B to 33D are horizontal cross-sectional views taken along lines B-B, C-C, and D-D of Fig. 33A, respectively.
Figs. 34A to 34C are schematic views illustrating a syringe-type spray/spout device in combination with a nasal spray/spout nozzle according to one embodiment of the present disclosure (Fig. 34A: perspective view, Fig. 34B: lateral plan view, and Fig. 34C: top plan view).
Figs. 35A to 35C are schematic views illustrating a syringe-type spray/spout device in combination with a nasal spray/spout nozzle according to one embodiment of the present disclosure (Fig. 35A: perspective view, Fig. 35B: lateral plan view, and Fig. 35C: top plan view).
Figs. 36A and 36B are schematic views illustrating a nasal rest article according to one embodiment of the present disclosure (Fig. 36A: perspective view, and Fig. 36B: top plan view).
Fig. 37 is a schematic perspective view illustrating a state wherein a nasal spray/spout nozzle according to the present disclosure has been inserted into a human nasal cavity for purpose of administration.
Fig. 38 is a schematic view illustrating a state wherein a nasal spray/spout nozzle according to the present disclosure has been inserted into a human nasal cavity, as viewed from an opening side of the external naris.
Fig. 39 is a schematic sectional view illustrating a state in which a nasal spray/spout nozzle according to the present disclosure has been inserted into a human nasal cavity for purpose of administration.
Fig. 40 is a schematic view illustrating an expansion of a medicament having ejected from a nozzle exit port.
Figs. 41A and 41B are schematic views illustrating a modified embodiment of a nose rest (Fig. 41A: top view, and Fig. 41B: lateral plan view).
Figs. 42A and 42B are schematic views illustrating a modified embodiment of a nose rest (Fig. 42A: top view, and Fig. 42B: lateral plan view).
Figs. 43A to 43C are images of nasal spray/spout nozzles used in Comparative examples (Fig. 43A: Comparative example 1, Fig. 43B: Comparative example 2, and Fig. 43C: Comparative example 3).
Figs. 44A to 44C are images of nasal spray/spout nozzles used in Examples (Fig. 44A: Example 1, Fig. 44B: Example 2, and Fig. 44C: Example 3).
Fig. 45 is an image of a nasal-cavity model used in a demonstration test.
Figs. 46A and 46B are schematic views of a position and an angle regarding a nozzle exit port in the demonstration test (Fig. 46A: an angle α, and Fig. 46B: an angle *β*).
Fig. 47 is a schematic sectional view of a human nasal cavity.

### MODES FOR CARRYING OUT THE INVENTION

### [Findings on which the Present Disclosure is based]

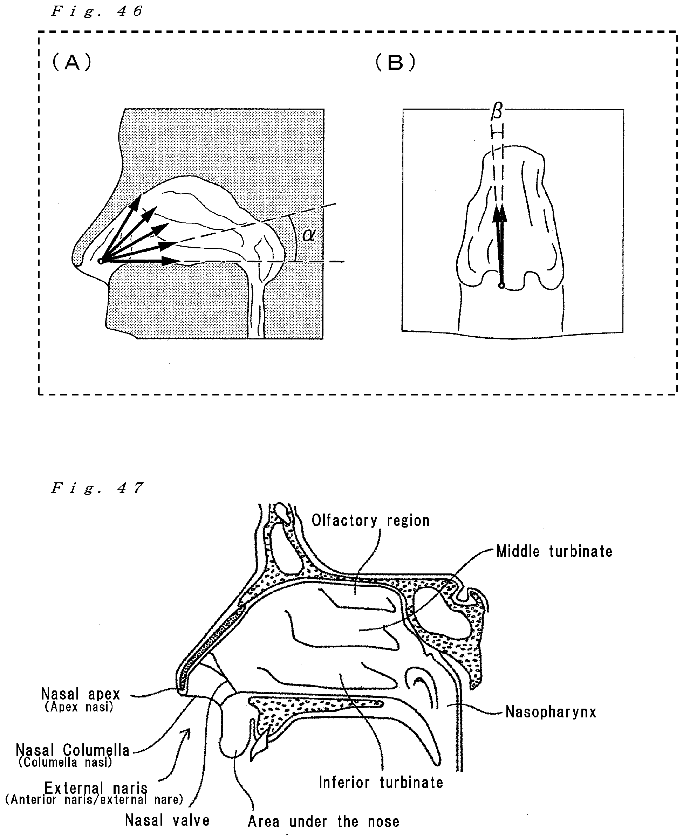

Some conventional spray/spout devices may not have a suitable configuration in that they are not suitably used for various complex intranasal structures. Regarding the conventional art, a nasal cavity having a narrower space around a nasal valve may be a factor causing a medicament to be unfavorably trapped so that the medicament may not be sufficiently delivered to a target region, for example (see Fig 47). The *"target region"* as used herein refers to a region at which a spray/spout aims with a nasal spray/spout nozzle. For example, the target region is such a region in the nasal cavity (i.e., intranasal region) as at least one selected from a nasal mucosa respiratory region, a nasopharynx region, and/or an olfactory region.

For example, when an insertion of the spray/spout nozzle of the nasal spray/spout device is excessively shallower in the nasal cavity, there is a possibility that the sprayed/spouted medicament is adversely trapped by the nasal valve or the like. On the other hand, when the insertion of the spray/spout nozzle is excessively deeper in the nasal cavity, a width of the medicament having ejected from the nozzle becomes adversely narrower with respect to the nasal target region so that there is a possibility that the medicament cannot be sufficiently delivered to a larger area of the nasal target region.

Therefore, the inventor has attempted to come up with a more suitable nasal spray/spout nozzle that is more suitably applicable to the complex intranasal structure.

Hereinafter, embodiments of the present invention will be described with reference to the accompanying drawings. The embodiments described below are intended to embody a basic technical concept of the present invention, and do not limit the present invention only to such embodiments unless otherwise specified.

In the drawings, some members having the same function as each other may be denoted by the same reference numeral. For suitable explanation of the main points of the present disclosure or easier understanding of the present invention, the embodiments may be separately described or shown, but a partial replacement or combination of the embodiments with each other is possible in terms of their configuration/structure. In the following embodiments, descriptions about matters that are common to those already described will be omitted, and in this case only difference(s) will be described. In particular, similar mechanisms and/or effects due to the similar configurations/structures will be omitted so as to avoid a duplicate explanation in each embodiment. The sizes, positional relationships, and the like of the member/portions illustrated in the drawings may be exaggerated for an improved clarity of explanation.

In the following description of the embodiments, terms regarding the directions (e.g., an "up-down direction", a "horizontal direction (left-right direction)", a "drawing-depth direction (perspective direction)", a "proximal side", a "distal side", and the like) are conveniently used for ease of understanding, and thus these terms are just for purpose of explaining the present disclosure so that the present disclosure is not adversely limited to them. In the accompanying drawings, similar portions/parts/members are illustrated by use of the same or similar reference numerals.

The "up-down direction", the "horizontal direction (left-right direction)", and the "drawing-depth direction (perspective direction)" as used herein correspond to those of drawings, and thus they correspond to an up-down direction, a horizontal direction, and a drawing-depth direction (perspective direction) in each of the drawings, respectively. For example, as for the "up-down direction", a downward direction in the verticality (i.e., a direction in which gravity acts) can refer to the "downward direction" / "lower", and also a direction opposite to the gravity-acting direction can refer to the "upward direction" / "upper".

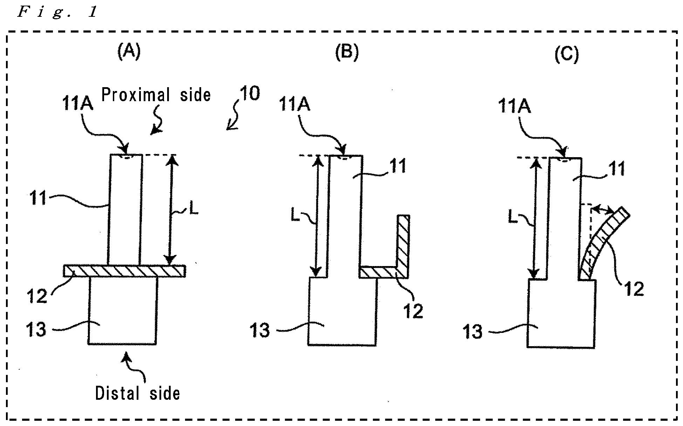

The term *"lateral plan view"* as used herein is based on a form/appearance when viewed in a direction that is approximately a perpendicular to the longitudinal direction of a nasal spray/spout nozzle. For example, the *"lateral plan view"* is based on forms/appearances as illustrated in Figs. 1A to 1C as well as other drawings. That is, the term *"lateral plan view"* is based on a form/appearance when the nasal spray/spout nozzle is laterally viewed from the outside in a direction that is approximately a perpendicular to the axis of the nasal spray/spout nozzle.

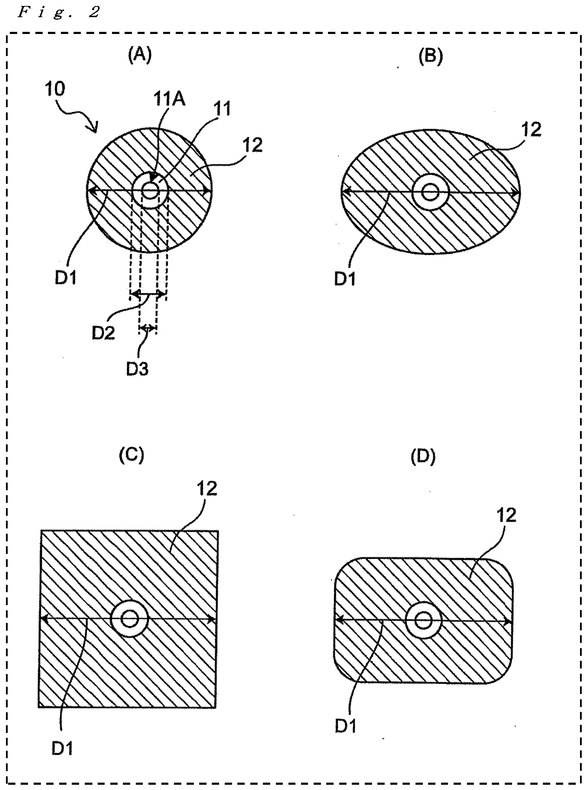

The term *"top plan view"* as used herein is based on a form/appearance when viewed from the side of a nozzle exit port of the nasal spray/spout nozzle. For example, the *"top plan view"* is based on forms/appearances as illustrated in Figs. 2A to 2D as well as other drawings. That is, the term *"top plan view"* is based on a form/appearance when the nasal spray/spout nozzle (the nozzle exit port thereof in particular) is viewed from the outside in the axial direction of the nasal spray/spout nozzle.

The term *"approximately"* as used herein refers to a state/form/shape that is close to an exact state/form/shape, or close to a complete state/form/shape as a whole. For example, the phrase *"approximately an elliptic shape"* is not necessarily limited to a shape recognized by those skilled in the art as an ellipse in a strict or exact sense, but can include shapes that are each recognizable by those skilled in the art as an ellipse in a non-strict or non-exact sense. For example, even in a case where a part of the contour of a certain shape constitutes the contour of another shape, the certain shape having such contour can also be regarded as an ellipse as long as it can be recognized as an elliptical shape as a whole by those skilled in the art.

The term *"diameter"* as used herein may mean a diameter of a circle, an ellipse, an inscribed circle or an inscribed ellipse as a shape/form viewed in a top plan view of an object. In this regard, the "inscribed circle" and the "inscribed ellipse" mean a circle and ellipse having the maximum area inside a polygon or the like, respectively. If a plurality of diameters are conceivable in the view, the longest diameter selected among such plurality of diameters can be regarded as the *"diameter"* in the present disclosure.

In some exemplary embodiments shown in the drawings, the term *"diameter"* may refer to the circle diameter D₁ in a case where the object is in a form of an exact circle (see Fig. 2A). Similarly, the term *"diameter"* may refer to the ellipse longest diameter D₁ in a case where the object is in a form of an ellipse (see Fig. 2B), the diameter D₁ of an inscribed circle in a case where the object is in a form of a square (see Fig. 2C), and also the longest diameter D₁ of an inscribed ellipse in a case where the object is in a form of a rectangle (see Fig. 2D).

### [Features of Nasal Spray/Spout Nozzle of the Present Disclosure]

The nasal spray/spout nozzle for nasal administration according to the present disclosure is a nozzle article for discharging a fluid material to the outside thereof. In particular, the nasal spray/spout nozzle of the present invention is a nozzle article for discharging the fluid material represented by a medicament, preferably with a form of spray and/or spout of the medicament.

The nasal spray/spout nozzle (or intranasal spray/spout nozzle) according to the present disclosure comprises a tip portion provided with a hole for ejecting as a nozzle exit port, and a nose rest capable of abutting on a nose region located around an external naris (i.e., a nose region located around *"anterior naris"* or *"external nare"*). In the exemplary embodiments shown in Figs. 1A to 1C, the nasal spray/spout nozzle 10 has the tip portion 11 provided with the nozzle exit port 11A thereof, and also the nose rest 12.

Examples of material for the nasal spray/spout nozzle according to the present disclosure may include, but not limited to, a resin material, a glass material, a metal material, a ceramic material, and the like. In a preferred embodiment, the nasal spray/spout nozzle corresponds to a resin product (e.g., a resin molded article).

The nasal spray/spout nozzle of the present disclosure may have a hollow portion in an interior or inside of the nozzle. For example, the hollow portion is provided in an interior or inside of the tip portion 11 in the nasal spray/spout nozzle 10 of the present disclosure. Such hollow portion is preferably in communication with the nozzle exit port. The hollow portion provided in the interior or inside of the nasal spray/spout nozzle may also be in communication with the outside at another nozzle side that is opposed to the side of the nozzle exit port. In other words, the nasal spray/spout nozzle may have its one end as an open end, the one end being opposed to the nozzle exit port in the axial direction of the nasal spray/spout nozzle.

As in a form of the nasal spray/spout nozzle illustrated in Fig. 1A as well as other drawings, the tip portion 11 and the nose rest 12 are connected to be adjacent to each other in the axial direction of the nasal spray/spout nozzle 10, for example. The "axis" as used herein refers to a virtual straight line passing through a center of the nasal spray/spout nozzle in a longitudinal direction of the nozzle.

In the nasal spray/spout nozzle 10, the tip portion 11 provided with the nozzle exit port, the nose rest 12, and a nozzle body 13 are located in such sequential order. Namely, the tip portion 11, the nose rest 12 and the nozzle body 13 are respectively provided in the nasal spray/spout nozzle from a nozzle side closer to the nose upon use of the nozzle. These parts/portions/members of the nozzle may each have a hollow portion in an interior or inside thereof. For example, the tip portion 11 may be a member/part having a hollow space in an interior thereof, in which case the tip portion 11 has a closed end provided with the nozzle exit port, and an open end that is opposed to the closed end. The nozzle body 13 may also be a member/part having a hollow space in an interior thereof, in which case the nozzle body 13 has open ends as its both ends that are opposed to each other. The nose rest 12 may be a member/part having, for example, a ring shape as a whole such that it has a hollow space inside thereof. Alternatively, the nose rest 12 may be a member/part having a partial ring shape such that it has a hollow space inside thereof with a part of the ring shape removed.

The tip portion 11 having the nozzle exit port, the nose rest 12, and the nozzle body 13 may be integrated with each other to provide the nasal spray/spout nozzle 10 (see Fig. 1A as well as other drawings). As can be seen from the forms of the nozzles shown in Fig. 1A as well as other drawings, the nose rest 12 may be regarded as a part, member or portion that is additionally provided with respect to the periphery of the tip portion 11 and/or nozzle body 13. The tip portion 11 and the nozzle body 13 may be regarded together as a single member/part in the nozzle such that they are in an integrated form with each other. For example, the single member/part, which in itself provides the tip portion 11 and the nozzle body 13, may be a cylindrical member/part having a hollow space in an interior thereof. In this regard, the nose rest 12 may be provided with respect to such single member/part of the tip portion and nozzle body. For example, a protruberance can be provided as the nose rest 12 on the outer peripheral face of the integrated single member/part of the tip portion 11 and nozzle body 13.

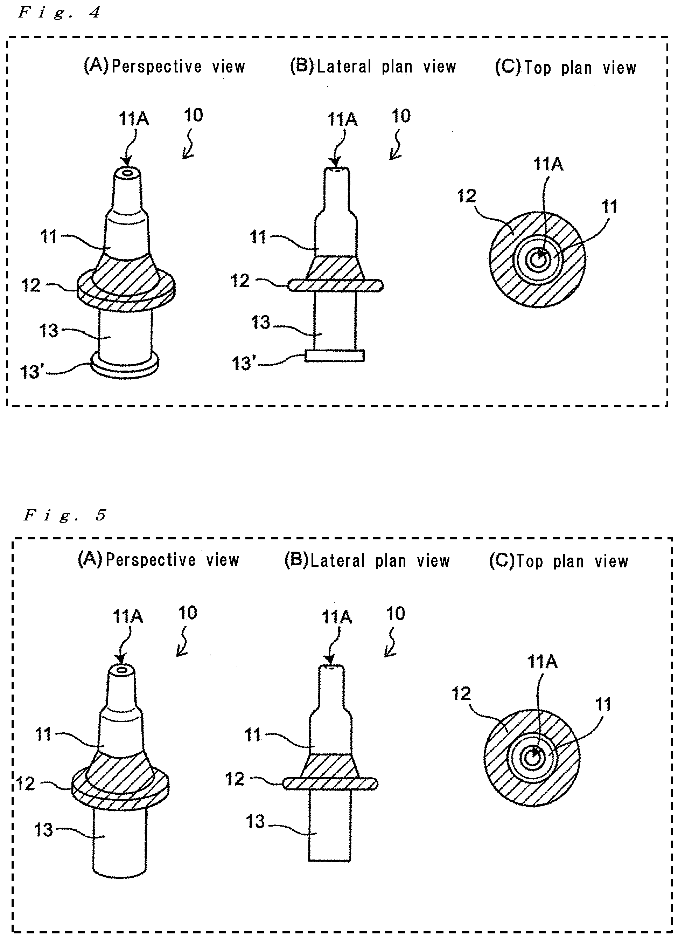

In the nasal spray/spout nozzle 10, the tip portion 11 extends from the nose rest 12 in the axial direction of the nasal spray/spout nozzle 10 (see Fig. 1A as well as other drawings). The nose rest 12 extends or protrudes outward such that it forms an angle with respect to the axis of the nasal spray/spout nozzle 10 (see Fig. 4 as well as other drawings). The *"outward"* as used herein corresponds to, for example, a radial direction that is approximately perpendicular to the axis of the nasal spray/spout nozzle 10.

At least a part of the nose rest 12 is capable of abutting on the nose region around the external naris. This makes it possible to stabilize the nasal spray/spout nozzle more suitably during its use such that a medicament delivery onto a target site of the nasal cavity can be improved. For example, the nasal spray/spout nozzle of the present disclosure can improve a delivery rate of the medicament delivered onto the nasal target site. While not wishing to be bound by any theory, such improved nozzle performance in terms of the medicament delivery can be due to a more suitable fixation or stabilization of an orientation of the nasal spray/spout nozzle during the use thereof, such as the more suitable fixation or stabilization of an angle of the nozzle (more specifically a nozzle angle formed with respect to a plane parallel or perpendicular to the nasal septum), and/or the more suitable fixation or stabilization of an insertion length of the tip portion of the nasal spray/spout nozzle into the nasal cavity.

In a preferred embodiment, the nose rest 12 can desirably restrict an inserted portion of the nasal spray/spout nozzle 10 into the nasal cavity to be almost only the tip portion 11. In other words, an insertion length of the nasal spray/spout nozzle 10, which is to be inserted into the nasal cavity, can be suitably set to be almost the same length as the length of the tip portion 11 (i.e., the length *"L"*). Thus, the nozzle exit port 11A can be appropriately positioned within the nasal cavity upon use of the nozzle. This means that an insertion depth regarding the nozzle exit port within the nasal cavity can be set as an appropriate one. Accordingly, with the nasal spray/spout nozzle of the present disclosure, the more suitable delivery of the medicament onto the target region can be achieved despite the fact that the nose has a complex intranasal structure. For example, by bringing at least a part of the nose rest into contact with the nose region located around the external naris, an inserted position of the nozzle exit port in the nasal cavity can be more appropriately set. Therefore, the medicament can be suitably delivered with the insertion of the nozzle being not excessively shallow or deep in the nasal cavity, which can lead to a facilitated delivery of the medicament to a larger area of the nasal target region.

The *"nozzle"* used herein is like what can define an orientation of a liquid substance, preferably what can define a flow direction of a medicament (e.g., a liquid agent or a gel agent), and thus is like what can discharge (e.g., spray and/or spout) the liquid substance such as the medicament from a hole provided at the tip thereof. Accordingly, it is preferred that the nozzle according to the present disclosure has a hollow portion in an interior thereof, and such hollow portion is in communication with a tip hole of the nozzle. The hollow portion of the nozzle can also be in communication with an open end of the nozzle. As such, the nozzle may be referred to also as a "spout" (or "spout article "/ "spout member"), a "member for spraying/spouting a medicament", a "member for spray/spout", or the like.

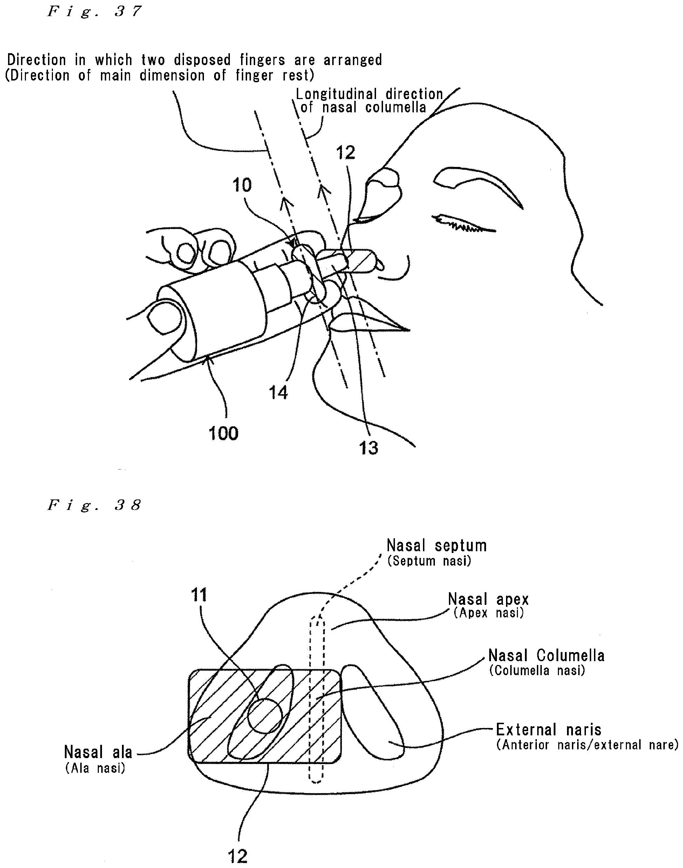

The *"nose region around an external naris"* as used herein refers to a nose area that is located around the external naris (see Figs. 38 and 47 especially as for the *"external naris"*). For example, the *"the nose region around an external naris" refers* to at least one selected from the group consisting of a nasal apex, a nasal columella, a nasal ala, an area under the nose (*"Shokuroku"*in Japanese that can refer to an area between nose and mouth), a wall surface of internal naris, and the like.

The term *"abut"* / *"abutting"* as used herein refers to a contacting preferably with a pressing mode. Examples of the *"abut"* / *"abutting"* as used herein include at least one selected from the group consisting of "contacting with a push mode", "contacting with a slide and subsequent stop mode", "contacting with a hook mode", "contacting with an engage mode" and the like.

The term *"tip portion"* as used herein refers to a portion of the nasal spray/spout nozzle, the portion being able to be inserted into a nasal cavity. The tip portion 11 may be positioned in the nasal spray/spout nozzle 10 at the proximal side of the nozzle. It is preferred that the tip portion 11 at least has its diameter that is smaller than the external naris (see Fig. 1A as well as other drawings).

In a top plan view of the nasal spray/spout nozzle 10, a width dimension of the tip portion 11 (e.g., the diameter D₂ as illustrated in Fig. 2A) may be 3 mm or more and 10 mm or less. The tip portion having the diameter D₂ of 3 mm or more can ensure a sufficient diameter for the nozzle exit port. While on the other hand, the tip portion having the diameter D₂ of 10 mm or less makes it easier to prevent the tip portion 11 from making an undesirable contact with the wall surface of the internal naris upon insertion of the nasal spray/spout nozzle into the nasal cavity. The diameter D₂ of the tip portion 11 is preferably 3 mm or more and 9 mm or less, and for example 3 mm or more and 7 mm or less.

The *"diameter D₂"* as used herein may be regarded as an average diameter of the tip portion 11, the average diameter being one obtained by averaging diameters in the axial direction of the nasal spray/spout nozzle 10. For example, the *"diameter D₂"* is an average value of diameters given at any three points of the tip portion 11 along the axis of the nozzle.

A diameter of a face having the nozzle exit port 11A (e.g., a diameter of a proximal end surface of the nasal spray/spout nozzle 10) may be the smallest in the tip portion 11. In this regard, the diameter of the face provided with the nozzle exit port 11A may be 3 mm or more and 10 mm or less, 3 mm or more and 9 mm or less, or 3.5 mm or more and 8.5 mm or less, for example. Such dimensions of the proximal end face can facilitate a smooth insertion of the tip portion upon inserting the nozzle into the nasal cavity, which can eventually lead to an improved performance of the nozzle in terms of the medicament delivery.

In a top plan view of the nasal spray/spout nozzle 10, a diameter of the nozzle exit port 11A (e.g., a diameter D₃ as illustrated in Fig. 2A) may be 0.15 mm or more and 0.60 mm or less. Setting the diameter D₃ within such range can facilitate enabling an ejection angle of the medicament from the nozzle to be more appropriate. The diameter D₃ of the nozzle exit port 11A is preferably 0.20 mm or more and 0.45 mm or less, and for example 0.25 mm or more and 0.30 mm or less.

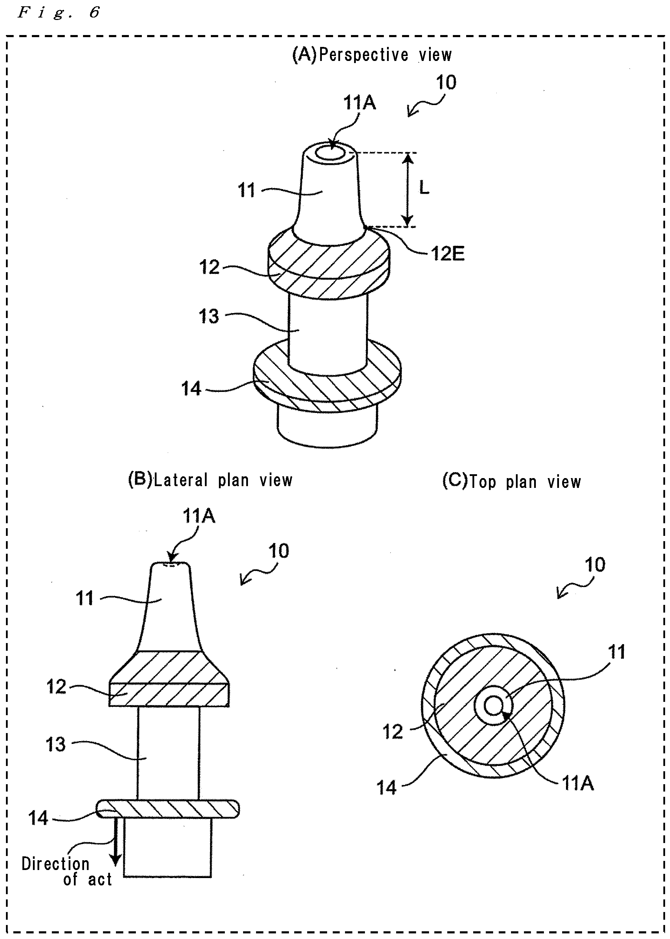

In a lateral plan view of the nasal spray/spout nozzle 10, the tip portion 11 has a length dimension "L" as shown in Fig. 1A. The *"length dimension L"* as used herein refers to a length dimension of the tip portion in the axial direction of the tip portion 11. That is, the *"length dimension L"*herein refers to a longitudinal dimension of the tip portion 11. More specifically, the *"length dimension* L"corresponds to a length from a proximal end of the nose rest 12 to the face provided with the nozzle exit port 11A. With reference to Fig. 6A, the *"length dimension L"*is a length extending from the tip end of the nasal spray/spout nozzle 10 (i.e., a nozzle end surface) to an end 12E of the nose rest 12 (i.e., the proximal end of the nose rest) in the axial direction of the nozzle.

In a lateral plan view of the nasal spray/spout nozzle 10, the length dimension L of the tip portion 11 in the axial direction of the nozzle may be 5 mm or more and 30 mm or less (see Fig. 1A as well as other drawings). The length dimension of 5 mm or more regarding the tip portion can facilitate reducing an undesirably trapped amount of the medicament to be trapped by the nasal valve or the like. The length dimension of 30 mm or less regarding the tip portion can facilitate an increase in the spray/spout width of the medicament to be ejected from the nozzle toward the target region, and thereby making it possible to suitably deliver the medicament over a larger area of the target region. The length dimension of the tip portion is preferably 7 mm or more and 25 mm or less, and for example 10 mm or more and 25 mm or less, 7 mm or more and 18 mm or less, 8 mm or more and 17 mm or less, 9 mm or more and 16 mm or less, or 10 mm or more and 15 mm or less.

The tip portion may have any form as long as it can be inserted into the nasal cavity. For example, the tip portion may have approximately a cylindrical shape. The tip portion may also have approximately a conical shape that is tapered toward the proximal end of the tip portion (i.e., approximately a conical shape that is tapered toward the nozzle exit port). The proximal end of the tip portion may be rounded so as to reduce a pain attributed to the contact of the tip portion with the wall surface of the internal naris. In this regard, such end of the tip portion may have a chamfered shape.

The term *"nose rest"* as used herein refers to a member/part provided as a portion of the nasal spray/spout nozzle, the portion being capable of abutting on the nose region around the external naris. More specifically, the nose rest refers to a member including a nozzle portion capable of abutting onto the nose region located around the external naris upon insertion of the nozzle into the external naris with the tip portion thereof being firstly inserted thereinto. The nose rest according to the present invention can be a member/part that protrudes or extends outward such that at least a part thereof is capable of abutting on the nose region around the external naris. The nose rest may be referred to also as a "nose-insertion stopper" or the like. It is preferred that the nose rest is a nozzle portion or member capable of abutting onto the nose region located around the external naris such that the inserted nasal spray/spout nozzle cannot be further inserted deeper into the nasal cavity.

The nose rest 12 may be positioned at the more distal side in the nasal spray/spout nozzle 10 than the tip portion 11 (see Fig. 1A as well as other drawings). That is, the nose rest 12 may be provided at a position farther from the nose holes than the tip portion 11 in the nasal spray/spout nozzle at a point in time when the nasal spray/spout nozzle is used. More specifically, as shown in certain figures of the present application, the nose rest 12 may be provided to be adjacent to the tip portion 11 while the nose rest 12 is positioned at the more distal side than the tip portion 11. The nose rest 12 may also be provided to be directly or indirectly connected to the tip portion 11 while the nose rest 12 is positioned at the more distal side than the tip portion 11. The nose rest may be a member or part integrated with the tip portion in the nasal spray/spout nozzle. Alternatively, the nose rest may be provided as a separate member or a separate part, in which case it can be attached to the tip portion so as to provide the nasal spray/spout nozzle. In a case where the tip portion and the nose rest are provided as an integrated member or part with each other, the tip portion and the nose rest may be made of the same material as each other, or may be made of different materials from each other.

The nose rest 12 may have any form as long as at least a part of the nose rest is capable of abutting onto the nose region around the external naris. That is, the nose rest 12 may have any form as long as it can make contact with the nose region located around the external naris so that they press against each other upon inserting the nasal spray/spout nozzle 10 into the nasal cavity, followed by further insertion of the nasal spray/spout nozzle 10 is inhibited due to such pressing against. In a preferred embodiment, only a part of the nose rest may be capable of pressing against the nose region located around the external naris. That is, the nose rest may protrude or extend outward such that only a part of the nose rest is capable of abutting onto the nose region located around the external naris. With only a part of the nose rest pressing against the nose region located around the external naris during use of the nozzle, an orientation and/or angle of the nasal spray/spout nozzle 10 can be facilitated to be suitably adjusted while a prevention of the further insertion of the nasal spray/spout nozzle 10 into the nasal cavity is suitably kept.

In one embodiment of the present disclosure, an outer contour of the nose rest 12 may surround at least a part of an outer contour of the tip portion 11 in a top plan view of the nasal spray/spout nozzle 10. For example, in a top plan view of the nasal spray/spout nozzle 10, a contour of the nose rest 12 (particularly an outer contour of the nose rest 12) is positioned to surround a contour of the tip portion 11 (particularly an outer contour of the tip portion 11). See Figs. 1A, 2A to 2D and 3A to 3D. For example, in the nasal spray/spout nozzle 10, the nose rest 12 may extend outward such that the nose rest has approximately a projecting shape or a flange shape.

Such configuration/form of the nasal spray/spout nozzle can facilitate a further increase in a contact area between the at least a part of the nose rest and the nose region located around the external naris at a point in time when the nozzle is used. Thus, the nasal spray/spout nozzle can be more firmly positioned with respect to the external naris during the use of the nozzle, and thereby the inserted position of the nozzle exit port can be more suitably ensured.

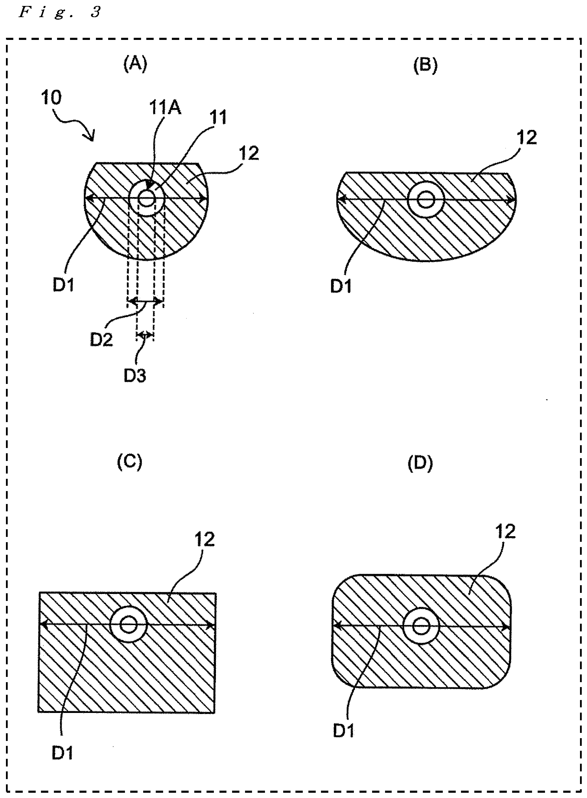

In a top plan view of the nasal spray/spout nozzle 10, the nose rest 12 may have approximately a circular shape, approximately an elliptic shape, a polygonal shape, a chamfered polygonal shape, or the like. With respect to each of approximately the circular shape, approximately the elliptic shape, the polygonal shape and the chamfered polygonal shape, the nose rest 12 may also have a modified shape in which a part thereof is cut out therefrom. Further, with respect to each of approximately the circular shape, approximately the elliptic shape, the polygonal shape and the chamfered polygonal shape, the nose rest 12 may also have such a modified shape that a nozzle position/axis is eccentric from the center of such shape. See Figs. 3A to 3D as well as Figs. 2A to 2D. From another standpoint, the nose rest may have approximately a flat plate shape, approximately a disk shape, approximately a dome-like shape, approximately a conical shape, approximately an elliptical cone shape, or other different shapes in a perspective view and/or lateral plan view of the nasal spray/spout nozzle.

In one embodiment of the present disclosure, the nose rest 12 may have approximately an elliptic shape in a top plan view of the nasal spray/spout nozzle 10 (see Fig. 2B). That is, in a top plan view of the nasal spray/spout nozzle 10, the nose rest 12 has such a shape that it largely protrudes in the two opposing directions. For example, the nose rest may have approximately a dome-like shape or approximately an elliptical cone shape.

Such configuration/form of the nasal spray/spout nozzle allows the nose rest to more easily conform to a shape/form of the nose region around the external naris during the use of the nozzle. This can further improve an abutting performance of the rest with respect to the nose region located around the external naris, and can also further improve a handling performance of the nasal spray/spout nozzle.

In a top plan view of the nasal spray/spout nozzle 10, a width dimension of the nose rest 12 (e.g., the diameter D₁ as illustrated in Figs. 2A to 2D) may be 10 mm or more and 50 mm or less. The diameter of 10 mm or more regarding the nose rest can facilitate the improvement of the abutting performance of the nose rest with respect to the external naris region. While on the other hand, the diameter of 50 mm or less of the nose rest can facilitate the improved handling of the nasal spray/spout nozzle 10. The diameter D₁ of the nose rest 12 may be 12 mm or more and 40 mm or less, and for example 15 mm or more and 30 mm or less.

The *"diameter D₁"* as used herein may be regarded as the longest width dimension of the nose rest 12, and for example the longest diameter of the nose rest 12. In this regard, the diameter D₁ may refer to a diameter of a circle, an ellipse, an inscribed circle, or an inscribed ellipse in a top plan view of the nose rest 12.

Alternatively, the nose rest 12 may have a shape capable of such abutment as if it can be hooked over the nasal region regarding the external naris. For example, the nose rest 12 may have an uneven shape, approximately a shape of "club with one claw" (*''Jitte''* in Japanese), approximately a shape of forceps, approximately a shape of a clip, or the like (see Figs. 1B and 1C).

Figs. 4 to 17 illustrate various embodiments of the nasal spray/spout nozzle according to the present disclosure. Specifically, Figs. 4 and 5 each illustrate the nasal spray/spout nozzle to be used for a syringe-type spray/spout device. Figs. 6 to 13 and Figs. 15 to 17 each illustrate the nasal spray/spout nozzle to be used for an upper exhaust airless-type spray/spout device.

In one embodiment of the present disclosure, the nose rest 12 may have a reducing portion that reduces a diameter of the nose rest 12 toward the nozzle exit port 11A (see Fig. 4B as well as other drawings). The nose rest may gradually reduce its diameter dimension toward the nozzle exit port, or may reduce its diameter dimension toward the nozzle exit port in a stepwise manner. In an exemplary embodiment shown in Fig. 4B as well as those of other drawings, the nose rest at least has a part/portion that gradually reduces its diameter dimension toward the nozzle exit port. For example, the nose rest at least has a part/portion that gradually reduces its width dimension as a whole. Such configuration/form of the nose rest can facilitate a further increase in the contact area between the nose rest and the nose region located around the external naris at a point in time when the nose rest abuts onto the nose region. In addition, when the nasal spray/spout nozzle 10 is inserted into the nasal cavity, it can be made possible for the reducing portion (i.e., the portion in which the reducing diameter of the nose rest is provided) to more suitably fit the wall surface of the internal naris.

That is, the nasal spray/spout nozzle 10 can more reliably press against the wall surface of the internal naris during the use of the nozzle due to the presence of the reducing portion of the nose rest 12. In this case, the inserted position and orientation of the nozzle exit port 11A are facilitated to be more suitably ensured. In addition, it is possible to mitigate an undesirable impact generated on the wall surface of the internal naris upon inserting the nasal spray/spout nozzle 10 into the nasal cavity, and thereby making it easier to reduce the discomfort such as a pain generated in a user (e.g., a patient).

In one embodiment of the present disclosure, the tip portion may at least have a taper part that is gradually tapered toward the nozzle exit port. For example, the tip portion 11 may have such a shape that it is gradually tapered toward the nozzle exit port 11A, as shown in Fig. 6B. Such configuration/form of the tip portion can facilitate preventing the tip portion 11 from making an undesirable contact with the wall surface of the internal naris upon inserting the nasal spray/spout nozzle 10 into the nasal cavity.

The tip portion 11, the nose rest 12 and/or the like may each have a lateral plan view in which the reducing diameter thereof can be recognized. For example, the tip portion 11, the nose rest 12 and/or the like may each have such a lateral plan view that the diameter thereof can be recognized to be reduced linearly (or increased linearly), the diameter thereof can be recognized to be reduced in a curved manner (or increased in the curved manner), the diameter thereof can be recognized to be reduced in a stepwise manner (or increased in the stepwise manner), or the diameter thereof can be recognized to be reduced in a mixed manner of linear and curve (or to be increased in the mixed manner of linear and curve). As for an exemplary embodiment shown in Fig. 6, a degree of reduction in diameter of the nose rest 12 (e.g., a degree of gradual reduction in diameter of the nose rest 12) may be larger than a degree of reduction in diameter of the tip portion 11. In other words, the nose rest 12 may form a smaller angle with respect to a plane perpendicular to the axis of the nasal spray/spout nozzle 10 than that of the tip portion 11.

In the nasal spray/spout nozzle 10 to be used in a syringe-type spray/spout device, the nozzle body 13 may be provided with a protrusion 13' for connecting with a syringe (see Fig. 4). Alternatively, the nozzle body 13 of the nasal spray/spout nozzle 10 may not be provided with such protrusion (see Fig. 5). When the improved handling is considered to some higher degree, it may be preferable to provide the nasal spray/spout nozzle 10 with the protrusion 13' for connecting with the syringe. A diameter dimension of the protrusion 13' is 5 mm or more and less than 15 mm, for example. As illustrated in Fig. 4, the protrusion 13' for connecting with the syringe may be provided at a lower end of the body of the nasal spray/spout nozzle 10. That is, the protrusion, which can serve for connecting with the syringe, may be provided at a distal end of the nasal spray/spout nozzle.

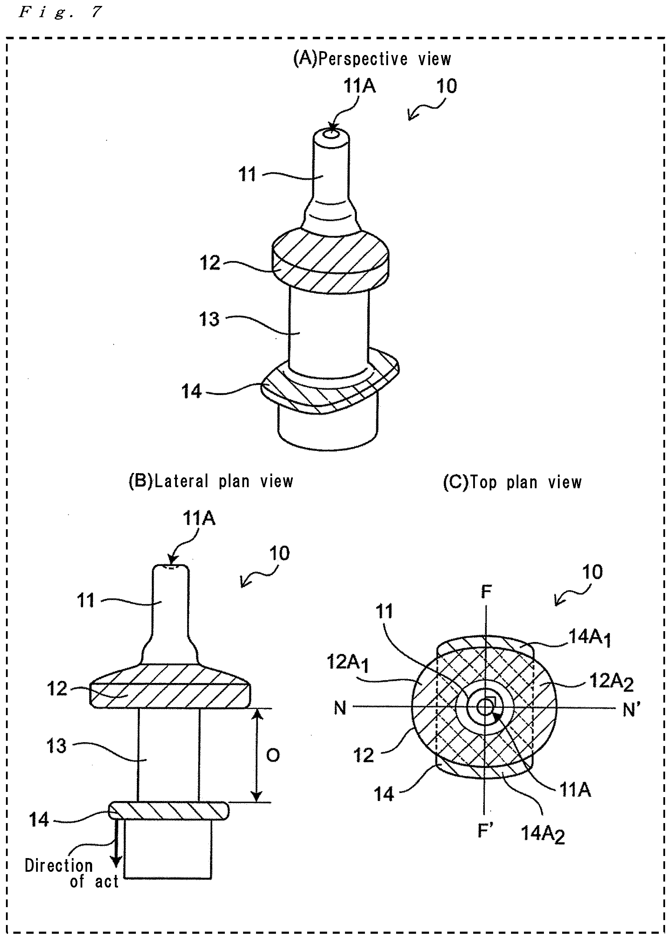

In one embodiment of the present invention, the nose rest 12 may have approximately an elliptic shape (see Fig. 7). That is, the nose rest 12 has approximately an elliptic shape in a top plan view of the nasal spray/spout nozzle. This configuration/form of the nose rest allows the nose rest 12 to more easily fit the nose region around the external naris (in particular, the nasal ala, the nasal columella, and/or the area under the nose) upon the insertion of the nasal spray/spout nozzle 10 into the nasal cavity.

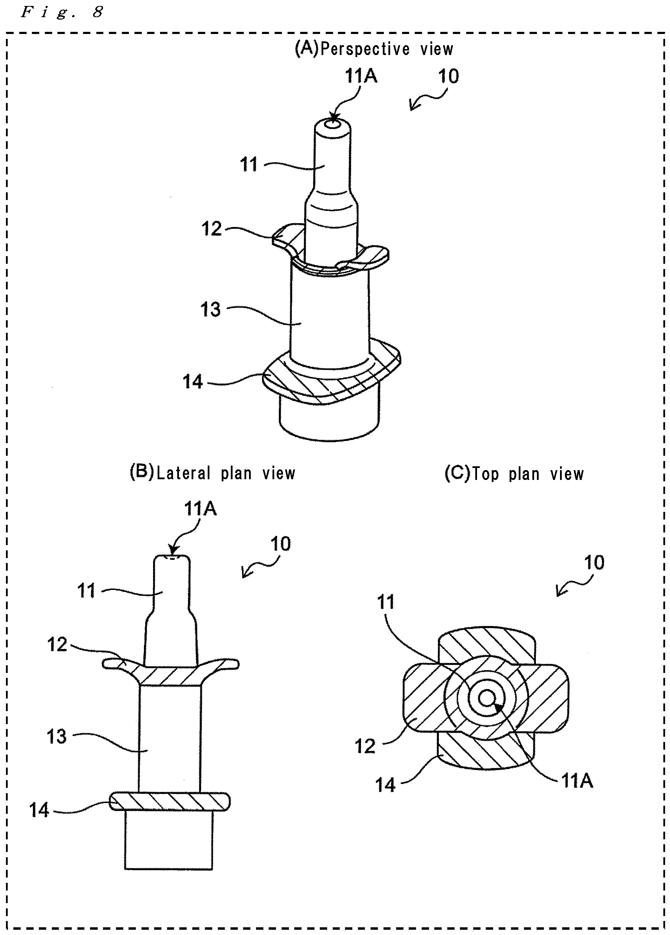

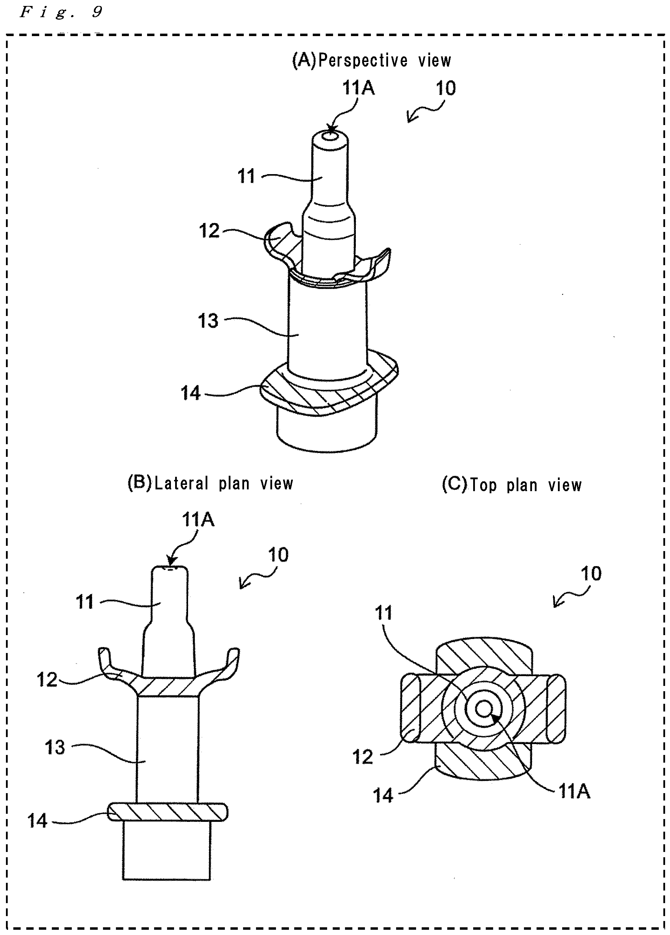

In one embodiment of the present invention, the nose rest 12 may have approximately a petal shape (see Figs. 8 and 9). When the nasal spray/spout nozzle 10 is inserted into the nasal cavity, this configuration/form of the nose rest 12 allows the nose rest to be more easily hooked over the ala of one nasal cavity into which the nozzle is inserted, and also hooked over the wall surface of the internal naris at the side of the other nasal cavity.

The tip of the nose rest 12 having approximately the petal shape may be curved/bent toward the nozzle exit port 11A (see Fig. 9). With this configuration/form of the nose rest, when the nasal spray/spout nozzle 10 is inserted into the nasal cavity, the nose rest 12 is further facilitated to be hooked over the ala of one nasal cavity into which the nozzle is inserted, and also hooked over the wall surface of the internal naris at the side of the other nasal cavity.

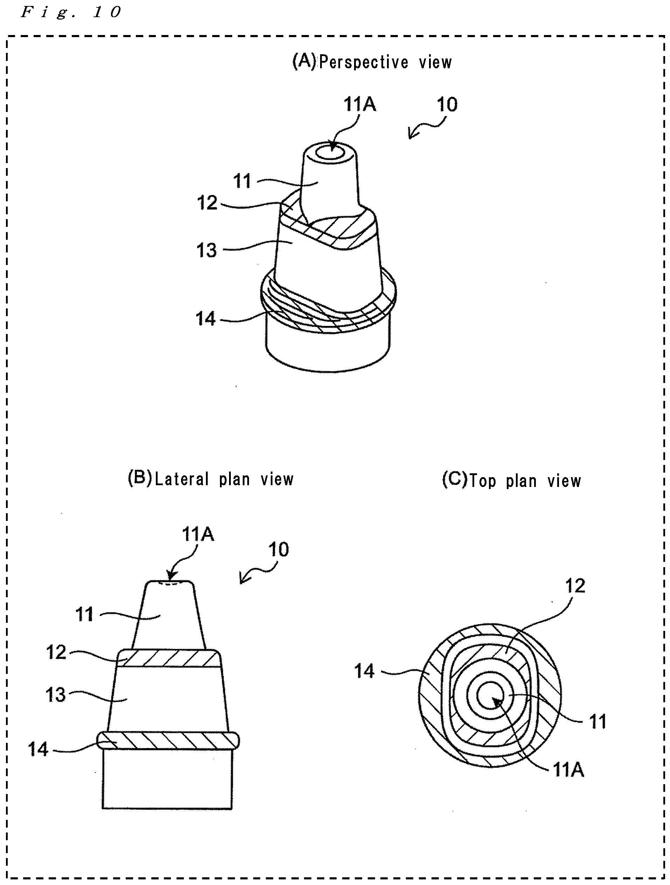

In one embodiment of the present disclosure, the nose rest 12 and the nozzle body 13 may not be in a step form (see Fig. 10). That is, an outer surface of the nose rest 12 and an outer surface of the nozzle body 13 may be integrally continuous to each other. This can mean that the outer surface of the nose rest 12 may be flush with the outer surface of the nozzle body 13. Such configuration/form regarding the nose rest and the nozzle body can simplify a manufacturing process of the nozzle. For example, the nasal spray/spout nozzle can be facilitated to be manufactured through an integral molding process.

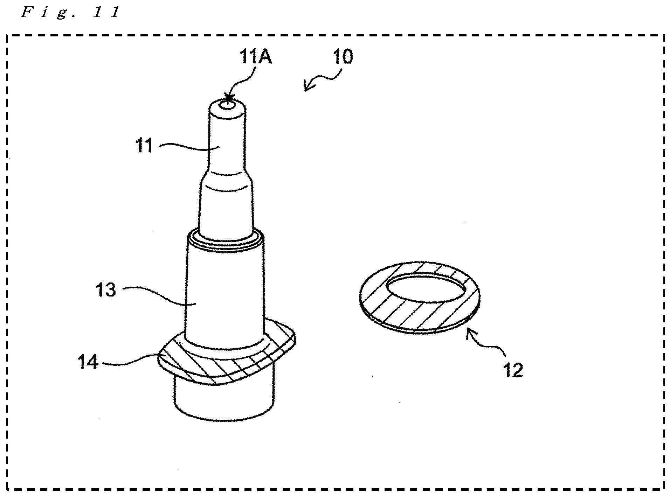

In one embodiment of the present disclosure, the nose rest 12 may be a member/part that is removable from the nasal spray/spout nozzle 10 (see Fig. 11 for example). That is, the nose rest 12 may be detachable from the nasal spray/spout nozzle 10 and/or attachable thereto. With this configuration/form of the nozzle, the nose rest 12 can be separately produced in manufacture of the nasal spray/spout nozzle 10. Further, with this configuration/form of the nozzle, the shape of the nose rest can be appropriately customized in accordance with each individual nose of users. The removable nose rest 12 may be made of a resin material. The removable nose rest 12 and the tip portion 11 may be made of different materials (e.g., different resin materials) from each other. In this case, for example, the nose rest 12 can be made of a material that has more cushioning characteristics. For example, the nose rest 12 can be made of a soft resin material or an elastomer material.

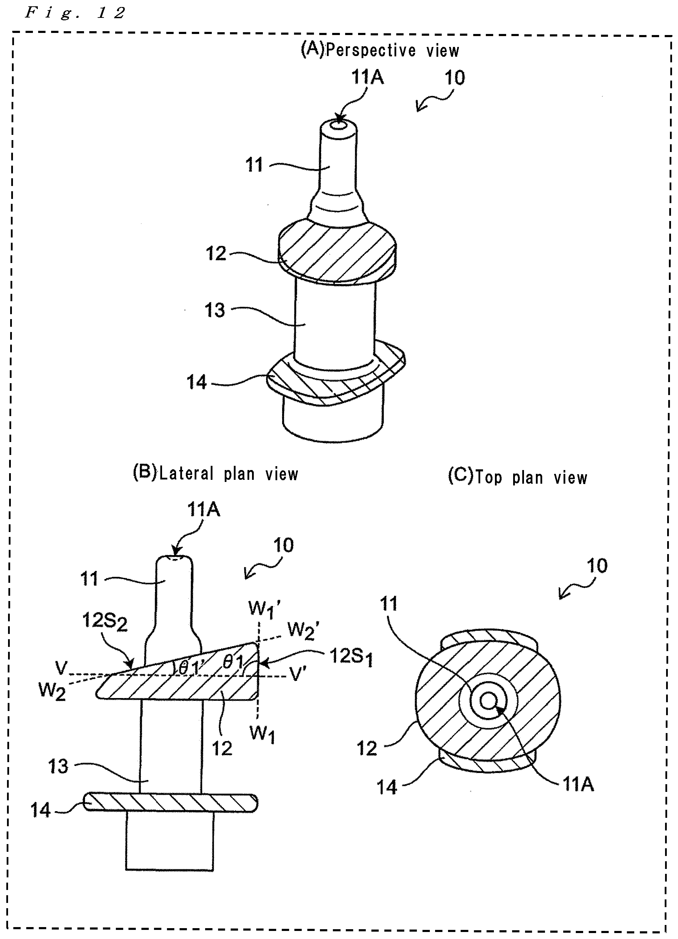

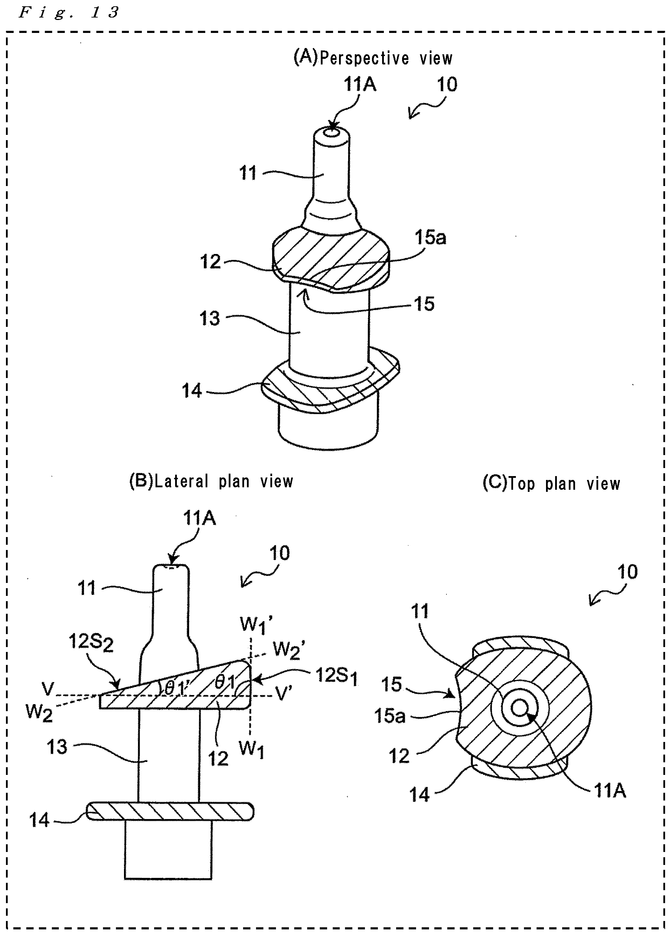

In one embodiment of the present disclosure, the nose rest may include a relatively steep face and a relatively non-steep face. For example, as shown in Figs. 12B and 13B, the nose rest 12 may have a reducing portion that reduces a diameter of the nose rest 12 toward the nozzle exit port 11A, and such reducing portion may include the relatively steep face and the relatively non-steep face. That is, a degree of inclination of a rest surface may be locally different in the nose rest.

The nose rest 12 may have its contour that gradually changes toward the tip portion 11 in a lateral plan view of the nasal spray/spout nozzle 10, in which case such contour may include a part where the gradual change is relatively large and also a part where the gradual change is relatively small (see Figs. 12B and 13B).

As for each of exemplary embodiments shown in Figs. 12B and 13B, the nose rest 12 includes a relatively steep face 12S₁ and a relatively gentle face 12S₂. More specifically, the inclined face 12S₁ forms an angle θ₁ with respect to a plane perpendicular to the axis of the nasal spray/spout nozzle 10 (i.e., with respect to the horizontal plane), and the inclined face 12S₂ forms an angle θ₁' with respect to such plane, i.e., the horizontal plane. That is, the angle θ₁ is formed by V-V' and W₁-W₁', and the angle θ₁' is formed by V-V' and W₂-W₂' in Figs. 12B and 13B. In this regard, the angle θ₁ may be larger than the angle θ₁'.

By allowing the inclined face 12S₂ or 12S₁ to abut onto the nose region around the external naris, the ejection direction/orientation (or angle) of the nozzle exit port can be adjusted for various target regions upon the insertion of the nasal spray/spout nozzle into the nasal cavity. In this regard, the angle θ₁' regarding the inclined face 12S₂ or the angle θ₁ regarding the inclined face 12S₁ (see Figs. 12B and 13B) may be adjusted in order for the ejection direction/orientation of the nozzle exit port 11A to be more suitable for various target regions.

Just as an example, the relatively gently inclined face 12S₂ as described above may be one capable of abutting onto a particular nose region of and/or near the area under the nose (i.e., onto the area under the nose, and/or onto a particular part of the nasal columella and/or the nasal alae, the particular part being located closer to the area under the nose), or may be one capable of abutting onto a particular nose region of and/or near a nasal apex (i.e., onto the nasal apex, and/or a particular part of the nasal columella and/or the nasal ala, the particular part being located closer to the nasal apex) See Fig. 47 as well as other drawings as for the *"area under the nose", "nasal apex", etc.*

In a case where the target region at which a spray/spout aims by the nasal spray/spout nozzle is a respiratory region or an olfactory region, the angle θ₁' may be for example 5° or more and 20° or less. For example, in a case where the target region is the respiratory region, it is preferable to allow the relatively gently inclined face 12S₂ to abut onto the particular region of and/or near the area under the nose. For example, in a case where the target region is the olfactory region, it is preferable to allow the relatively gently inclined face 12S₂ to abut onto the particular region of and/or near the nasal apex.

In one embodiment of the present disclosure, the nose rest 12 may have a difference in its local thickness. For example, due to the relatively steep inclined face, the relatively non-steep inclined face and the like, the nose rest 12 may have both a relatively thin portion and a relatively thick portion. This can mean that the nose rest 12 does not have a point-symmetrical thickness feature from the plan viewpoint of the nasal spray/spout nozzle 10.

By allowing the nose rest having such difference in its local thickness to abut onto the nose region around the external naris, the ejection direction/orientation (or angle) of the nozzle exit port is facilitated to be controlled for various target regions upon the insertion of the nasal spray/spout nozzle into the nasal cavity. In this regard, such local thickness difference of the nose rest may be adjusted in order for the ejection direction/orientation of the nozzle exit port to be more suitable for various target regions.

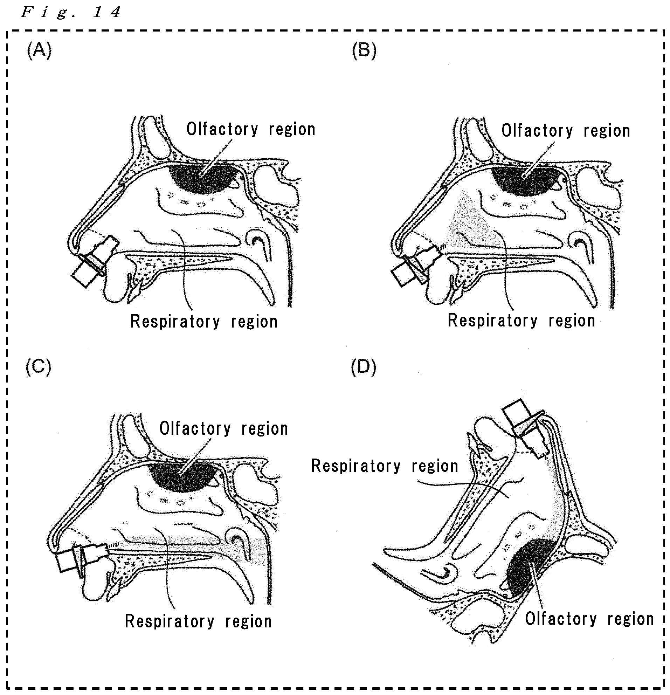

That is, when the relatively thin portion or relatively thick portion of the nose rest 12 is allowed to abut onto the nose region around the external naris, the target region for which the spray/spout is performed by the nasal spray/spout nozzle can be more desirably set due to a relationship between the "thin or thick portion" of the nose rest and a "contact region of the thin or thick portion of the nose rest with the nose region located around the external naris" (see Figs. 14A to 14D, particularly Figs. 14B to 14D). Just as an example, when the relatively thin portion of the nose rest 12 is allowed to abut onto the area under the nose or the periphery of thereof, the nasal spray/spout nozzle can be more easily inserted in its orientation closer to the perpendicular to the horizon. This makes it possible for the olfactory region to be preferably set as the target region at which the spray/spout aims by the nasal spray/spout nozzle. On the other hand, when the relatively thick portion of the nose rest 12 is allowed to abut onto the area under the nose or the periphery thereof, the nasal spray/spout nozzle can be more easily inserted in its more horizontal orientation, i.e., in a more parallel orientation with respect to the ground. This makes it possible for the nasopharynx region to be preferably set as the target region at which the spray/spout aims by the nasal spray/spout nozzle.

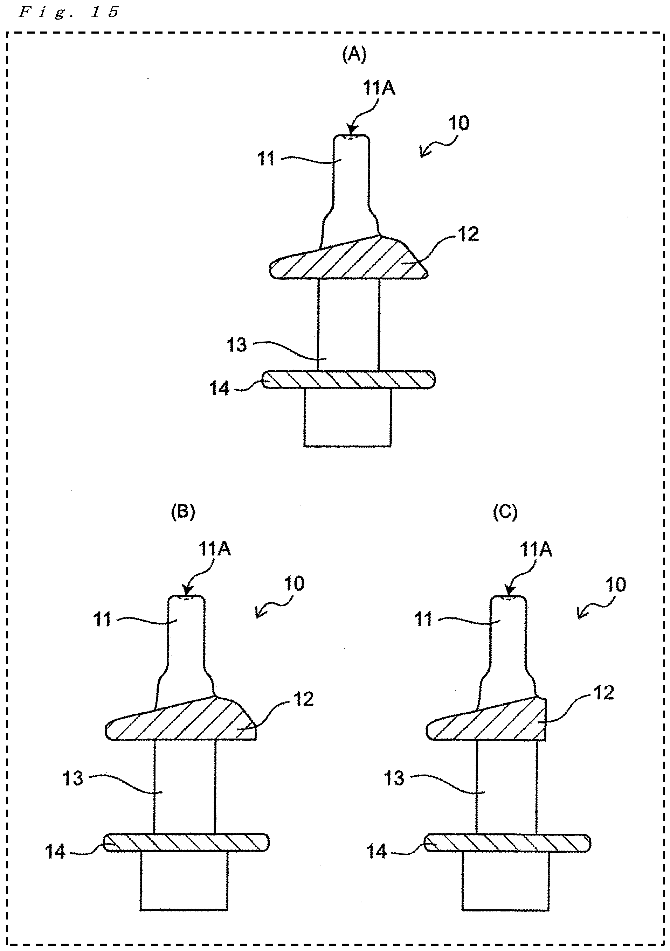

The nose rest 12 with its difference in local thickness can take various forms. For example, the nose rest 12 with its difference in local thickness can have each of forms as shown in Fig. 15. That is, the nose rest 12 with its local thickness difference may have such a form that a part of the contour of the nose rest in a lateral plan view of each of Figs. 12 and 13 has been removed. More specifically, the nose rest 12 with its local thickness difference may have such a form that at least a part of the contour of the relatively thick portion in a lateral plan view has been removed from the contour as illustrated in each of Figs. 12 and 13. With the nose rest as shown in Fig. 15, the nasal spray/spout nozzle can be more suitably inserted into the nasal cavity. Alternatively, with the nose rest as shown in Fig. 15, the relatively thin portion of the nose rest can be allowed to more suitably abut onto the desired nose region around the external naris (see Figs. 14C and 14D).

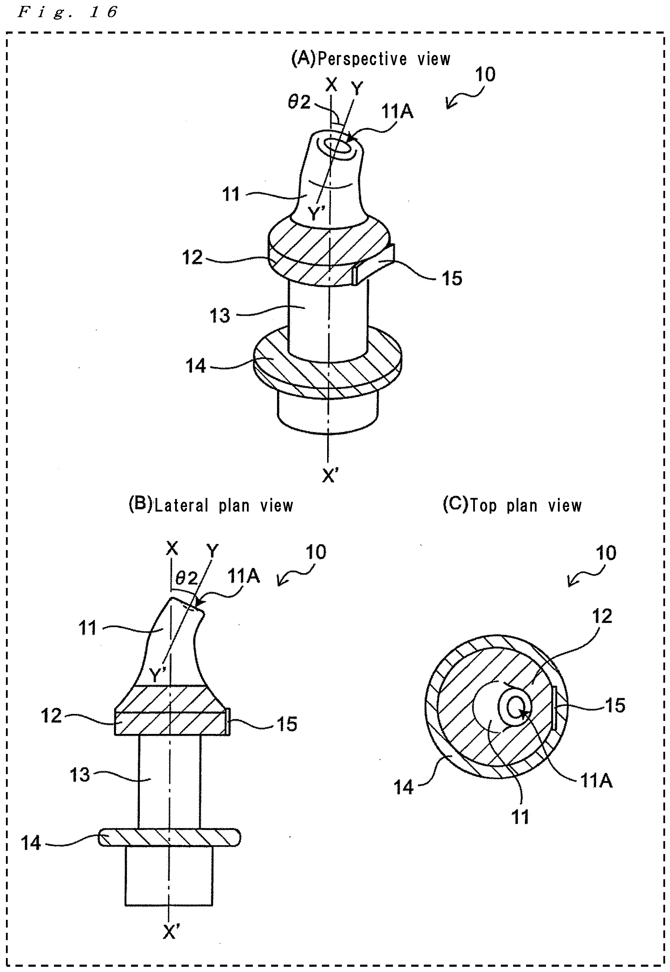

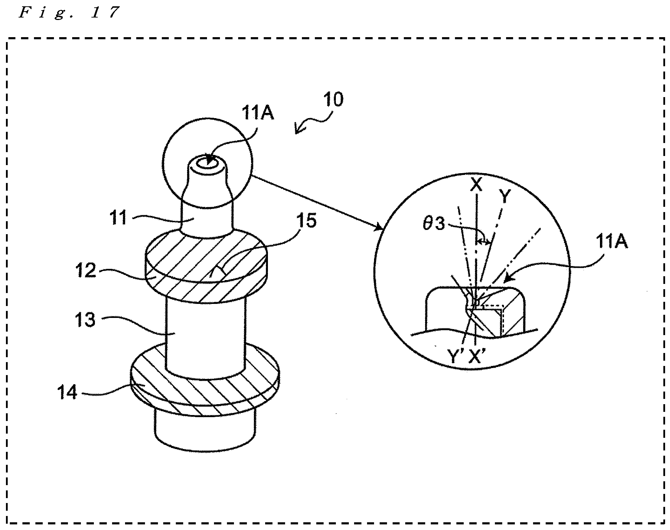

In one embodiment of the present disclosure, an ejection direction of the nozzle exit port 11A may form an angle θ₂ or θ₃ with respect to the axis of the nasal spray/spout nozzle 10 (see Figs. 16 and 17). More specifically, an axis X-X' of the nasal spray/spout nozzle 10, and a normal line Y-Y' to a nozzle plane that defines the nozzle exit port 11A (i.e., the ejection direction of the nozzle exit port 11A) may form such angle θ₂ or θ₃.

The normal line Y-Y' to the nozzle plane which defines the nozzle exit port 11A may form the angle θ₂ with respect to the axis X-X' of the nasal spray/spout nozzle 10 due to a curved or bent tip portion 11 (see Figs. 16A and 16B). The *"curved"* or *"bent"* used herein may refer to an embodiment wherein the tip portion is already in the curved or bent form. Alternatively, the *"curved"* or *"bent"* used herein may refer to another embodiment wherein the tip portion is capable of being curved or bent at any time for use of the nozzle.

Alternatively, the normal line Y-Y' to the plane which defines the nozzle exit port 11A may form the angle θ₃ with respect to the axis X-X' of the nasal spray/spout nozzle 10 by an oblique arrangement of the nozzle exit port 11A itself (see Fig. 17).

Each of the angles θ₂ and θ₃ (see Figs. 16B and 17) may be defined such that it is given by the nozzle axis and a particular direction in which an abutment part of the nose rest 12 to be able to abut onto the nose region located around the external naris faces (e.g., the direction in which the abutment part 15 of the nose rest faces in Fig. 16A). In other words, the angles θ₂ and θ₃ may be each defined with respect to the up-down direction of Figs. 16B and 17. This makes it easier to suitably deliver the medicament to the target region having various intranasal structures.

**In** the above embodiment, the angles θ₂ and θ₃ are each preferably 5° or more and 15° or less in a case where the target region at which the spray/spout aims by the nozzle is the respiratory region or the olfactory region. Especially in a case where the target region is the respiratory region, it is preferable to allow a part of the nose rest, which part is positioned closer to an opening direction/ejection direction of the nozzle exit port (e.g., closer to the right-sided part of the nose rest in Fig. 16B), to abut onto the nose region around the external naris. While on the other hand, especially in a case where the target region is the olfactory region, it is preferable to allow a part of the nose rest, which part is positioned closer to the side opposite to the opening direction/ejection direction of the nozzle exit port (e.g., closer to the left-sided part of the nose rest in Fig. 16B) to abut onto the nose region around the external naris.

In a case where the target region is the nasopharynx region, the angles θ₂ and θ₃ may be each 5° or more and 15° or less. In this regard, for example, it is preferable to allow a part of the nose rest, which part is positioned closer to the opening direction/ejection direction of the nozzle exit port, to abut onto the nose region around the external naris.

Each of the angles θ₂ and θ₃ (see Figs. 16B and 17) may be defined such that it is given by the nozzle axis and a particular direction that is approximately perpendicular to the direction in which an abutment part of the nose rest 12 to be able to abut onto the nose region located around the external naris faces. In other words, the angles θ₂ and θ₃ may be each defined with respect to the drawing-depth direction of Figs. 16B and 17. This also makes it easier to suitably deliver the medicament to the target region having the intranasal structure. In particular, it is possible to more suitably prevent the medicament from being adversely ejected from the nozzle toward the nasal septum or alar.

In such embodiment, the angles θ₂ and θ₃ may be each 5° or more and 15° or less. In this regard, it is preferable to allow a part of the nose rest, which part is positioned closer to the direction approximately perpendicular to the opening direction/ejection direction of the nozzle exit port (e.g., closer to the drawing depth-sided part of the nose rest in Fig. 16B), to abut onto the nose region around the external naris.

In order to prevent a misuse of the nozzle, the nasal spray/spout nozzle may have a fit surface or a directive marker. As for the fit surface, it may be provided to fit the nose region around the external naris upon the insertion of the nasal spray/spout nozzle into the external naris. The fit surface 15 or the directive marker 15 may be provided at any position of the nasal spray/spout nozzle 10. For example, the fit surface 15 or the directive marker 15 may be provided in the nose rest 12 (see Figs. 13, 16, and 17).

In one embodiment of the present invention, the fit surface may be in a form of a notch (i.e., a cutout form) or in a form of a contact plate. In other words, the nose rest may have the notch or plate for contacting or fitting the nose region located around the external naris.

The notch may be in any form as long as it can fit the nose region around the external naris (e.g., the nasal columella, the nasal ala, or the area under the nose). For example, the notch 15 may serve as adapting to the shape/form of the nasal ala, the nasal columella, or the area under the nose (see Fig. 13).

For example in a case where the nose rest has approximately an elliptic shape in a top plan view, the nose rest with the notch being provided may have such a shape that a part of a contour of approximately the elliptical shape has been cut out as compared with an original shape as the ellipse. Examples of a surface provided by the notch (e.g., a surface 15a in Fig. 13) in the nose rest may include a flat surface and/or a curved surface. For example, the "notch" may correspond to having a form of a "concave", a "U-shaped portion" or the like.

The contact plate may also be in any form as long as it can fit the nose region around the external naris. For example, the contact plate 15 may serve as adapting to the shape/form of the nasal ala, the nasal columella, or the area under the nose (see Fig. 16).

The *"contact plate"* may itself have a concave shape, a U-shape or the like, for example. Due to the presence of the contact plate, a part of the outer contour of the nose rest may be provided as the concave contour, the U-shaped contour or the like. As the contact plate, a separate member (e.g., a member made of an elastomer material) may be attached to the nose rest.

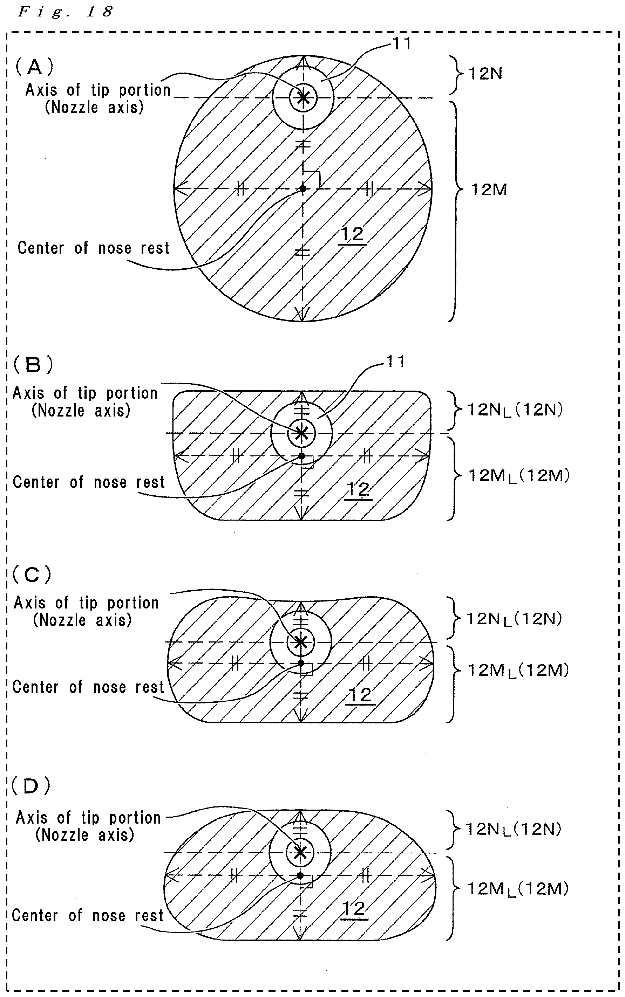

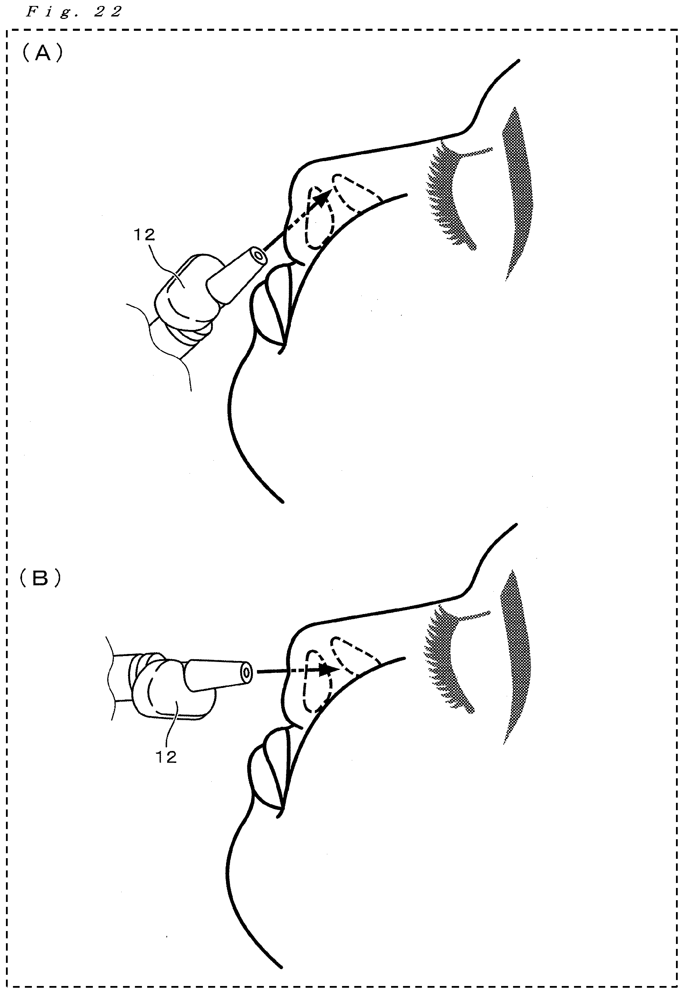

In one embodiment of the present disclosure, in a top plan view of the nasal spray/spout nozzle, an axis of the tip portion 11 and a center of the nose rest 12 have an eccentric relationship with each other such that the axis of the tip portion 11 and the center of the nose rest 12 are offset from each other (or such that they are not aligned with each other). See Figs. 18A to 18D. That is, the nose rest may be provided to be eccentric with respect to the axis of the nasal spray/spout nozzle. The *"center"* as used herein may be, for example, a barycenter (e.g., gravity center) of the nose rest. For example, this center of the nose rest may be a point corresponding to the middle point of each of the two width dimensions that are perpendicular to each other in a top plan view (see Figs. 18A to 18D). In this embodiment, a rest part at one side of the nose rest 12 located around the tip portion 11 may extend relatively wider or larger than that of the other side. For example, as shown in Figs. 18A to 18D, a rest region at one side of the nose rest 12 may extend relatively wider or larger than a rest region at the other side of the nose rest 12, the one side and the other side being opposed to each other (or faced with each other). As for the embodiments shown in Figs. 18B and 18C, in a top plan view of the nasal spray/spout nozzle, a longer-sided region 12M_{L}, which extends at one of longer sides of the nose rest, may be larger or wider than a longer-sided region 12N_{L}, which extends at the other of longer sides of the nose rest, the longer-sided region 12M_{L} and the longer-sided region 12N_{L} being opposed to each other across a virtual line where the axis of the tip portion 11 passes. This means that one region of the nose rest and the other region thereof may be different in size from each other, the one region and the other region facing each other across the tip portion. Such size may correspond to a size of a flattened portion of the nose rest, for example. As for each of exemplary embodiments shown in Figs. 18A to 18D, a wider portion 12M having a relatively large width dimension and a narrower portion 12N having a relatively small width dimension are provided in the nose rest. In such nose rest, a desired orientation/angle of the tip portion can be more easily ensured when the nasal spray/spout nozzle is allowed to abut onto the nose region around the external naris. That is, the orientation/angle characteristics of the nasal spray/spout nozzle during the use thereof can be improved. For example, when the nose rest is used such that the wider portion 12M of the nose rest is oriented as an upper side of the nose rest (e.g., such that the wider portion 12M of the nose rest becomes closer to the nasal apex), the nasal spray/spout nozzle can be more easily disposed in such a manner that the tip portion of the nose rest becomes more erected, that is, in such a manner that the angle "α" of Fig. 46A becomes larger. See Fig. 22A. This can lead to a facilitated spray/spout of the medicament onto an upper nasal region in the nasal cavity (e.g., the olfactory region). On the other hand, when the nose rest is used such that the wider portion 12M of the nose rest is oriented as a lower side of the rest (e.g., such that the wider portion 12M becomes closer to the area under the nose), the nasal spray/spout nozzle can be more easily disposed in such a manner that the tip portion becomes laid further, that is, in such a manner that the angle "α" of Fig. 46A becomes smaller. See Fig. 22B. This can lead to a facilitated spray/spout of the medicament onto a lower or deeper nasal region of the nasal cavity (e.g., the nasopharynx region).

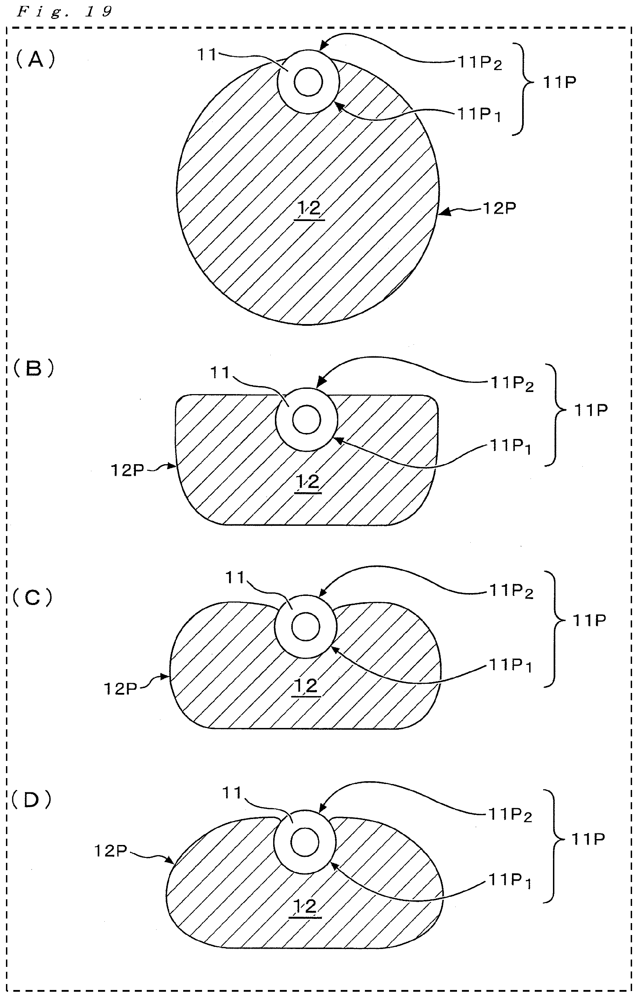

In one embodiment of the present disclosure, an outer contour of the tip portion has a non-surround part in which a part of the outer contour of the tip portion is not surrounded by an outer contour of the nose rest in a top plan view of the nasal spray/spout nozzle. That is, the nose rest may be provided to be more greatly eccentric with respect to the axis of the nasal spray/spout nozzle. In this embodiment, as shown in Figs. 19A to 19D, not the whole of an outer contour 11P of the tip portion 11 is surrounded by an outer contour 12P of the nose rest 12, and thus a non-surround part 11P₂ is present at only one location of the outer contour 11P of the tip portion 11. In this case, the outer contour 11P of the tip portion may be composed of a surround part 11P₁ and the non-surround part 11P₂. With such configuration/form of the nozzle, the orientation/angle characteristics of the nasal spray/spout nozzle during the use thereof can be more suitably improved.

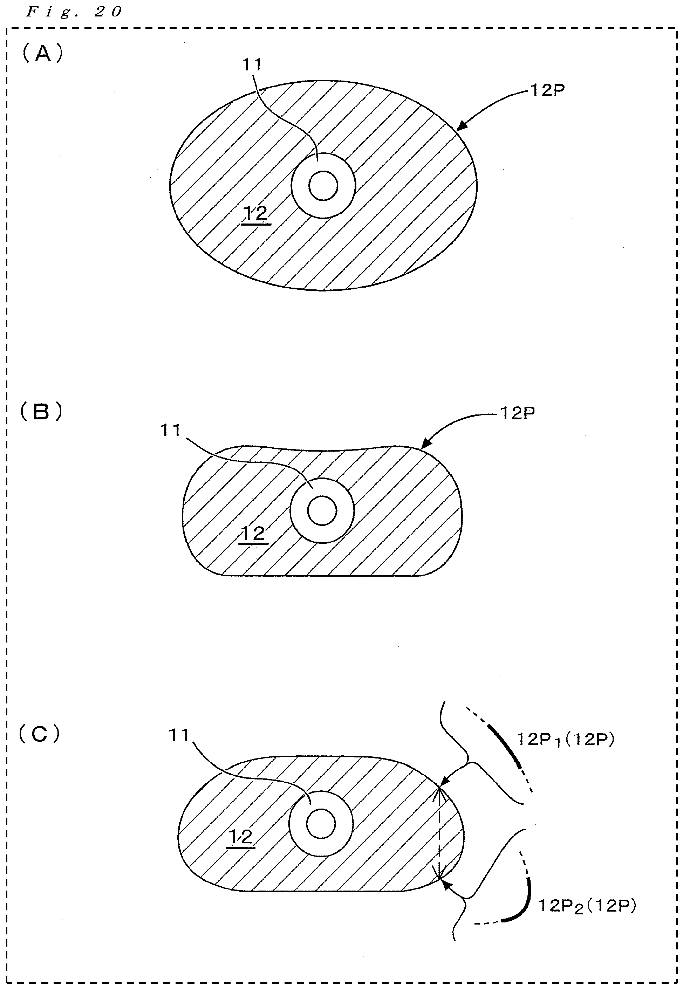

In one embodiment of the present disclosure, in a top plan view of the nasal spray/spout nozzle, the outer contour of the nose rest may be in a form of arc at one side of the nose reset. As shown in Figs. 20A to 20C, at least a part of the outer contour 12P of the nose rest 12 may be a rounded or curved contour, not a linear contour. Fig. 20 A shows an exemplary embodiment of the nose rest in which all of the outer contour 12P thereof is in a form of arc, and Figs. 20B and 20C each show an exemplary embodiment of the nose rest in which a part of the outer contour 12P thereof is in a form of arc. A part of the outer contour of the nose rest at one side thereof may have different in curvature from another part of the outer contour of the nose rest at the other side thereof that is opposed to the one side. For example, as for an exemplary embodiment of Fig. 20C wherein the nose rest has a part 12P₁ at one side of the outer contour 12P thereof and a part 12P₂ at the other side of the outer contour 12P thereof, the one side and the other side facing each other, the part 12P₁ at the one side has a relatively smaller curvature than that of the part 12P₂ at the other side. This means that the part 12P₁ at the one side of the outer contour of the nose rest may be in a form of a gentler curve than that of the part 12P₂ at the other side of the outer contour of the nose rest. With the outer contour of the nose rest having such form of arc, the orientation/angle characteristics of the nasal spray/spout nozzle during the use thereof can be more suitably improved. The shape of the nose rest may be not angular in a top plan view. If the nose rest is angular in a top plan view, the angular portion of the nose rest may undesirably affect the nose of the user during the use of the nasal spray/spout nozzle. Accordingly, when the outer contour of the nose rest has the arc form at least at one side thereof, the orientation/angle of the nasal spray/spout nozzle during the use thereof can be facilitated to be changed or controlled more suitably than that of the nose rest with the angular form.

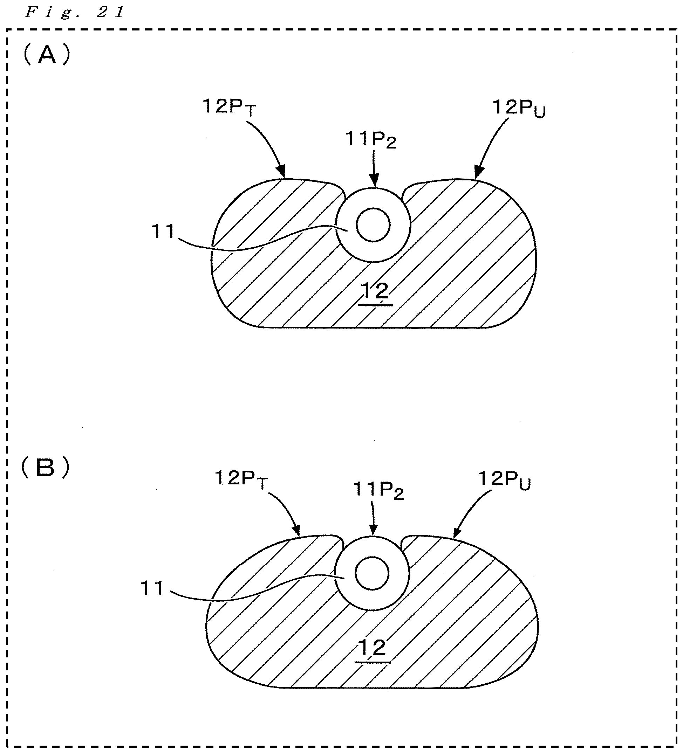

The arc form of outer contour of the nose rest may be combined with the eccentric feature(s) as described above. For example, as shown in Figs. 21A and 21B, in a top plan view of the nasal spray/spout nozzle, parts 12P_{T} and 12P_{U} situated at both sides of the non-surround part 11P₂ in the outer contour of the nose rest may be each in a form of arc. This can promote the effect of easily changing the orientation/angle of the nasal spray/spout nozzle during the use thereof. In particular, as illustrated in Fig. 21B, the nose rest's outer contour parts 12P_{T} and 12P_{U} situated at both sides of the non-surround part 11P₂ of the tip portion may be in a form of elliptical arc. Further, the parts 12P_{T} and 12P_{U} of the outer contour of the nose rest, which are located at both sides of the non-surround part 11P₂ of the tip portion, may be in such a symmetrical form that they are symmetrical to each other about the non-surround part 11P₂ in a top plan view. The nose rest 12 as a whole in a top plan view may have a shape of a "pea" (see Fig. 21B), for example. These can further promote the effect of easily changing the orientation/angle of the nasal spray/spout nozzle during the use thereof.

An exemplary use mode regarding the nose rest as shown in Fig. 21B is illustrated in Figs. 22A and 22B. As illustrated in these Figures, the nose rest 12 can be reversible for use in accordance with the target region such that the orientation of the nose rest is reversed. The use mode of the nozzle in Fig. 22A makes it possible for the medicament to be more suitably delivered onto the upper nasal region in the nasal cavity (e.g., the olfactory region). On the other hand, the use mode of the nozzle in Fig. 22B makes it possible for the medicament to be more suitably delivered onto the lower or deeper nasal region in the nasal cavity (e.g., the nasopharynx region).

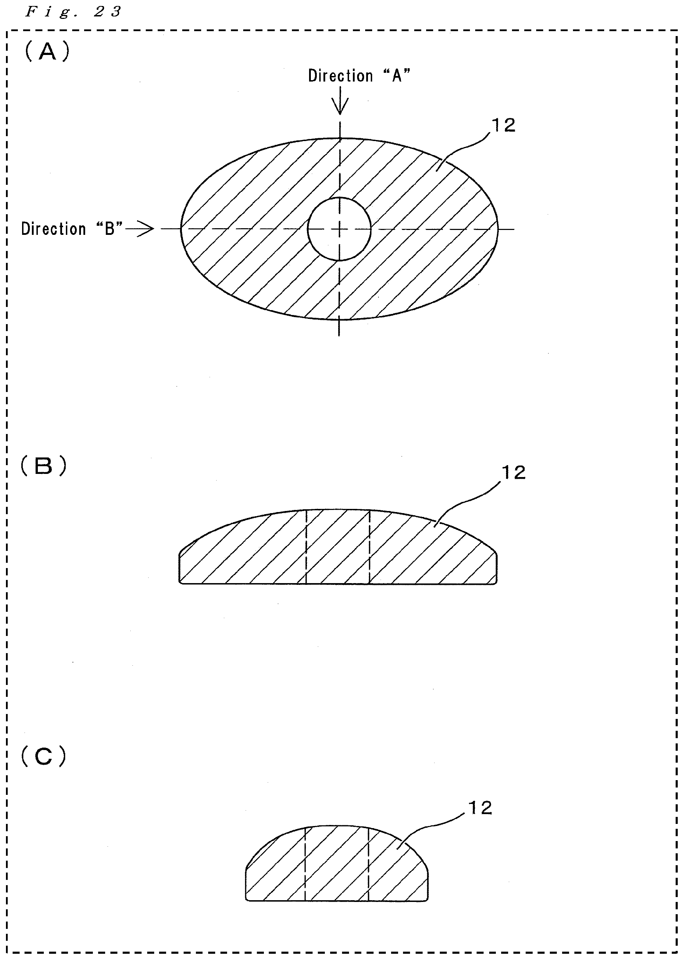

In one embodiment of the present disclosure, a surface of the nose rest, which is provided especially at the proximal side of the nozzle, may be in a form of dorm. The nose rest 12 may have such a shape as illustrated in Figs. 23A to 23C (particularly Figs. 23B and 23C). The nose rest 12 may be no angular in a lateral plan view, and thus the surface of the nose rest 12 at the proximal side may be wholly in a curved form. This embodiment allows the orientation/angle of the nasal spray/spout nozzle to be easily changed during the use of the nozzle, and also can reduce a discomfort of the user, the discomfort being attributed to the resistance for the nose rest.

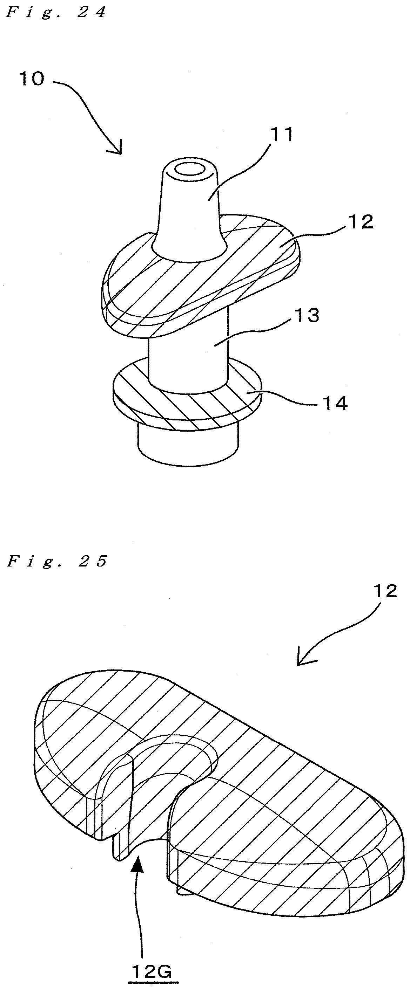

Fig. 24 shows the nasal spray/spout nozzle 10 according to one exemplary embodiment of the present invention. The nose rest 12 provided in the nasal spray/spout nozzle 10 of Fig. 24 has the above features. For one thing, the axis of the tip portion 11 and the center of the nose rest 12 have an eccentric relationship with each other such that the axis of the tip portion and the center of the nose rest are offset from each other (or such that they are not aligned with each other), and the outer contour of the tip portion 11 has the non-surround part in which a part of the outer contour of the tip portion is not surrounded by the outer contour of the nose rest 12. The surface of the nose rest 12 at the proximal side has a dome shape. In a top plan view of the nose rest 12, the nose rest's contour parts 12P_{T} and 12P_{U} situated at both sides of the non-surround part 11P₂ of the outer contour of the tip portion are in a form of elliptical arc (see Fig. 21B). Such nose rest 12 may be integrated with the tip portion 11 and/or the nozzle body 13 in advance. Alternatively, the nose rest 12 may be attachable to and/or detachable from the tip portion 11 and/or the nozzle body 13. Fig. 25 shows an example of such removable nose rest 12. The nose rest 12 may be attached to the tip portion 11 and/or the nozzle body 13, for example, in a snap-fit manner. Specifically, the nose rest 12 may be attached to the tip portion and/or the nozzle body and/or may be detached from the tip portion and/or the nozzle body in the lateral direction of the nose rest 12 through a lateral opening 12G of the nose rest 12.

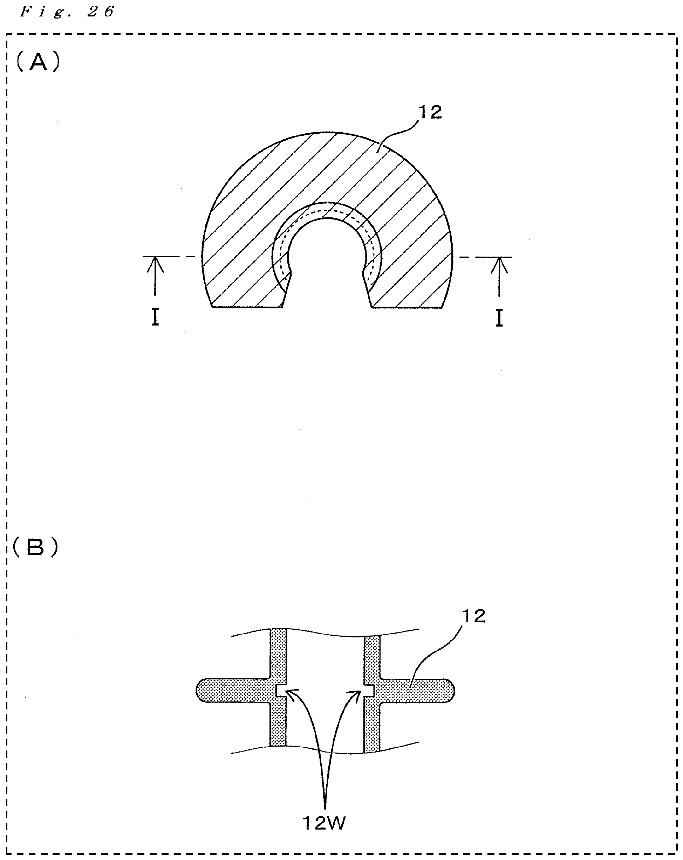

Various embodiments are possible as for the removal nose rest 12. The nose rest 12 may be attached to provide the nasal spray/spout nozzle by allowing the nose rest to be mounted from the proximal end of the tip portion. This is particularly conceivable when the removable nose rest 12 does not have the lateral opening 12G shown in Fig. 25. As shown in Fig. 26B, a recess 12W may be provided in an inner wall of the nose rest 12. While on the other hand, a projection capable of complementarily engaging with the recess 12W may be provided in the tip portion 11 and/or the nozzle body 13. With an engagement (e.g., fitting)/disengagement of the recess 12W and the projection with each other, the nose rest 12 can be attached to and/or detached from the tip portion 11 and/or the nozzle body 13. Such recess and the projection can be reversed with respect to each other. That is, the projection may be provided in the inner wall of the nose rest 12, whereas the recess may be provided in the tip portion 11 and/or the nozzle body 13.

In one embodiment, the nasal spray/spout nozzle 10 may further comprise a finger rest 14 for putting a finger on the finger rest (see Figs. 6 and 7 as well as other drawings). In other words, the nasal spray/spout nozzle 10 may include a first increased-diameter portion 12 due to the nose rest, and also a second increased-diameter portion 14 due to the finger rest.

In a broader sense, the term *"finger rest"* as used herein refers to a portion for putting a finger thereon during use of the nasal spray/spout nozzle. In a narrower sense, the term *"finger rest"* as used herein refers to a portion for putting a finger on such portion when the nasal spray/spout nozzle is inserted into the nasal cavity. From this viewpoint, the finger rest may be referred to also as a "finger hook", a "finger grip" or the like. In one embodiment of the present invention, the finger rest is positioned at the more distal side in the nasal spray/spout nozzle 10 than the nose rest. For example, in the nasal spray/spout nozzle 10, the second increased-diameter portion 14 serving as the finger rest and the first increased-diameter portion 12 serving as the nose rest are spaced away from each other in the axial direction of the nozzle. In this regard, the second increased-diameter portion 14 may be positioned at the more distal side in the nozzle than the first increased-diameter portion 12.

The finger rest of the nasal spray/spout nozzle can further improve the handling performance of the nozzle in terms of the spraying/spouting of the medicament.

The finger rest may have any form as long as it allows a finger to be put on the finger rest. For example, an outer contour of the finger rest 14 may surround at least a part of the outer contour of the tip portion 11 in a top plan view of the nasal spray/spout nozzle 10 (see Fig. 6C as well as other drawings). In such configuration/form of the nozzle, the finger can be suitably put on the finger rest 14 such that the finger can be more easily acted in an act direction of the nasal spray/spout nozzle 10 (see Fig. 6B). The finger rest may be a member/part having its constant thickness, for example.

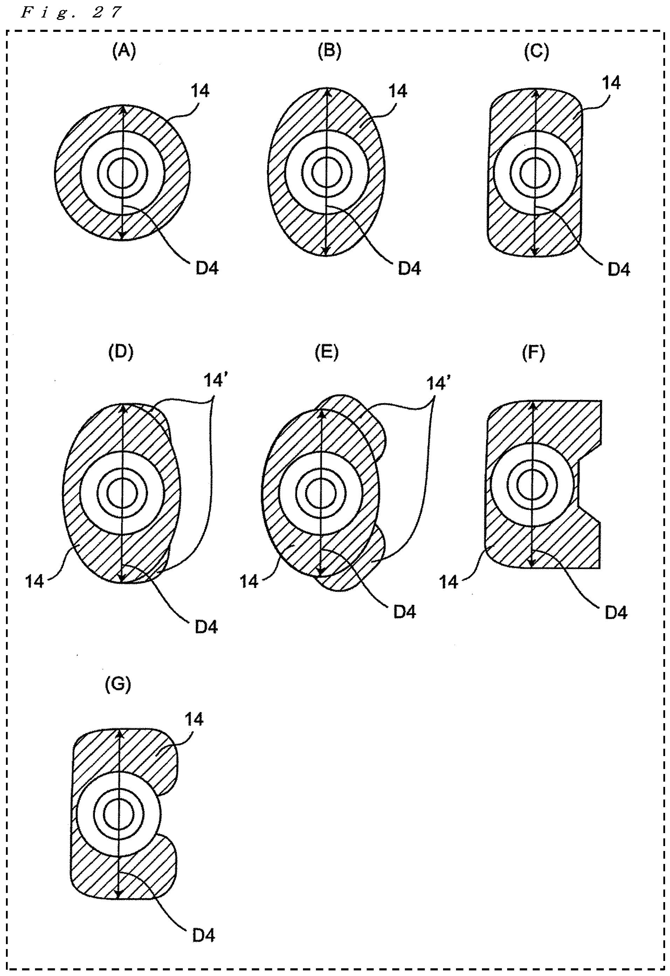

In a top plan view of the nasal spray/spout nozzle 10, the finger rest 14 may have approximately a circular shape, approximately an elliptic shape, a polygonal shape, a chamfered polygonal shape, or the like (see Figs. 27A to 27G).

In terms of placing more importance on the handling of the nozzle, the finger rest 14 may have approximately an elliptic shape or approximately a rectangular shape in a top plan view (see Figs. 27B and 27C). The finger rest 14 may be provided with a finger guide 14' (see Figs. 27D and 27E). Such finger rest can facilitate positioning a finger more reliably on the finger rest, and thereby making it easier to suitably ensure the inserted position and orientation of the nozzle exit port. The finger rests of Figs. 27A to 27E are just examples. Thus, the finger rest 14 may have, as a top plan view, other forms as illustrated in Figs. 27F and 27G, for example.

In a top plan view of the nasal spray/spout nozzle 10, a width dimension of the finger rest 14 (e.g., a diameter D₄ as illustrated in Figs. 27A to 27G) may be 15 mm or more and 60 mm or less. The diameter of 15 mm or more can facilitate an improved efficiency in hooking of the finger over the finger rest. While on the other hand, the diameter of 60 mm or less can further improve the handling performance of the nasal spray/spout nozzle 10. The diameter D₄ of the finger rest 14 is preferably 17 mm or more and 50 mm or less, and for example 20 mm or more and 45 mm or less.

The "*diameter D₄"* as used herein may be regarded as the longest diameter of the finger rest 14. The diameter D₄ in this regard may refer to a diameter of a circle, an ellipse, an inscribed circle, or an inscribed ellipse in a top plan view of the finger rest 14. In a case where the finger rest 14 has the finger guide 14' (see, for example, Figs. 27D and 27E), the diameter D₄ may be regarded as a diameter of a circle, an ellipse, an inscribed circle, or an inscribed ellipse in a virtual shape obtained by removing the finger guide 14' from the finger rest 14.

In one embodiment of the present disclosure, a diameter N-N' of the nose rest 12 and a diameter F-F' of the finger rest 14 are approximately perpendicular to each other in a top plan view of the nasal spray/spout nozzle 10 (see Fig. 7C). This configuration/form of the nozzle can further facilitate suitably ensuring the orientation of the nozzle exit port 11A in the nasal cavity.

The "*approximately perpendicular"* as used herein means that it does not necessarily have to be exact 90°. For example, the *"approximately perpendicular"* may be in the range of 90° ± 10°. In the illustrated form of Fig. 7C, the diameter N-N' of the nose rest 12 and the diameter F-F' of the finger rest 14 form an angle of 90° therebetween.

In the above embodiment, the longest diameter N-N' of the nose rest 12 and the longest diameter F-F' of the finger rest 14 may be approximately perpendicular to each other in a top plan view of the nasal spray/spout nozzle 10. Specifically, the nose rest 12 and the finger rest 14 may each have approximately an elliptic shape or approximately a rectangular shape as a shape of a top plan view, in which case the longest diameter of the nose rest and the longest diameter of the finger rest may be approximately perpendicular to each other.

The above feature can mean that the nose rest 12 and the finger rest 14 may form approximately a cross shape in a top plan view of the nasal spray/spout nozzle 10 (see Fig. 7C). It is preferred that the longest diameter N-N' and the longest diameter F-F' form approximately a cross shape so as to be line-symmetric. That is, the longest diameter N-N' and the longest diameter F-F' preferably intersect with each other at each center thereof.

In other words, the nose rest 12 and the finger rest 14 have an overlap portion with each other in a top plan view of the nasal spray/spout nozzle 10, while an opposing direction of the two non-overlapping portions 12A₁ and 12A₂ regarding the nose rest 12 and an opposing direction of the two non-overlapping portions 14A₁ and 14A₂ regarding the finger rest 14 may be approximately perpendicular to each other (see Fig. 7C).

Such configuration/form of the nozzle can facilitate positioning the nasal spray/spout nozzle with respect to the external naris more easily and more accurately. That is, it can be made particularly easier to suitably ensure the orientation of the nozzle exit port 11A within the nasal cavity.

In one embodiment of the present disclosure, the axis of the nose rest 12 and the axis of the finger rest 14 may be aligned with each other in a top plan view of the nasal spray/spout nozzle 10 (see Fig. 7C as well as other drawings). In other words, the nose rest 12 and the finger rest 14 may have an identical axis in the nasal spray/spout nozzle 10. Such configuration/form of the nozzle can further facilitate improving the handling performance of the nasal spray/spout nozzle 10.

In one embodiment of the present disclosure, the spaced distance "O" between the nose rest 12 and the finger rest 14 may be 10 mm or more and 50 mm or less, and for example 10 mm or more and 30 mm or less in a lateral plan view of the nasal spray/spout nozzle 10 (see Fig. 7B). In other words, a length dimension "O" of the nozzle body 13 (or a part of the nozzle body 13) in the nasal spray/spout nozzle 10 may be 10 mm or more and 50 mm or less, and for example 10 mm or more and 30 mm or less.

Such dimension "O" of 10 mm or more can facilitate preventing the nose rest from interfering with a finger upon putting the finger on the finger rest. While on the other hand, the dimension "O" of 50 mm or less can facilitate improving the handling performance of the nozzle in terms of the spraying/spouting of the medicament.

The spaced distance between the nose rest and the finger rest may be different depending on the users in terms of whether the nasal spray/spout nozzle is used for self-use or for the other person. In a case where the nasal spray/spout nozzle is used for the self-use, it is preferred that the spaced distance "O" between the nose rest and the finger rest is 15 mm or more and 30 mm or less, and for example 15 mm or more and 25 mm or less. While on the other hand, in a case where the nasal spray/spout nozzle is used for the other person, the spaced distance "O" between the nose rest and the finger rest may be 50 mm or more and 70 mm or less.

### (Nasal Spray/Spout Device)

Examples of a nasal spray/spout device using the nasal spray/spout nozzle according to the present disclosure may include a conventional spray/spout device, and also an upper exhaust airless-type spray/spout device and a syringe-type spray/spout device. Such nasal spray/spout device can comprise the nasal spray/spout nozzle incorporated thereinto. Now, the upper exhaust airless-type spray/spout device and the syringe-type spray/spout device will be described in detail.

### (Upper Exhaust Airless-type Spray/Spout Device)

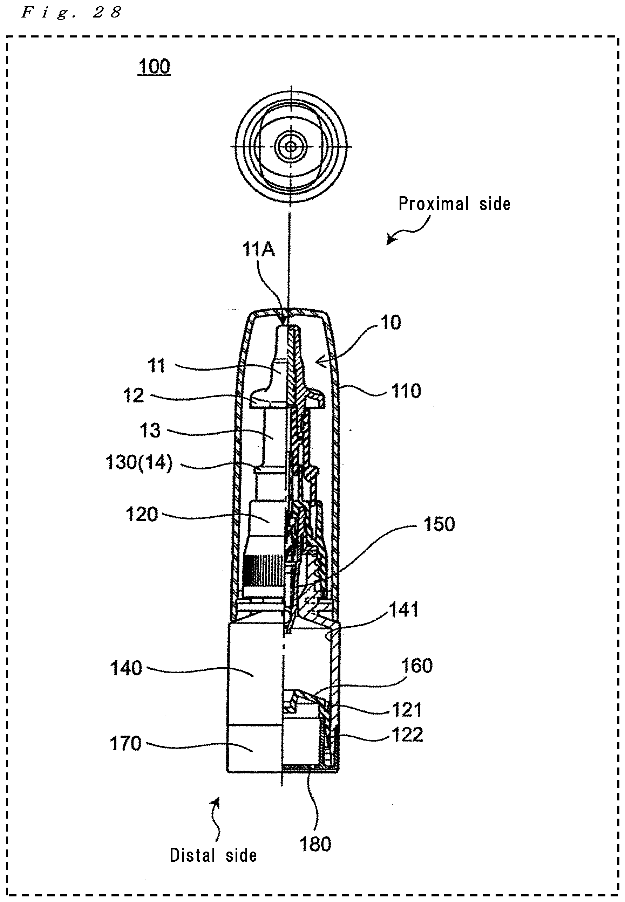

Fig. 28 illustrates an embodiment of an upper exhaust airless-type spray/spout device 100 provided with the nasal spray/spout nozzle 10 according to one embodiment of the present disclosure. Such airless-type device 100, which is capable of an upward-direction exhaustion, has a protective cap 110 to be removed prior to use of the device. At a point in time after the protective cap 110 is removed, the upper exhaust airless-type spray/spout device 100 is capable of ejecting a content of a container 140 to the outside through an exit hole by pushing down an annular operating portion 130 provided for the pump 120. For such ejection, the content is sucked into a suction chamber 150 of the pump 120 from the inside of the container 140. The annular operating portion 130 is provided at the head of the spray/spout device 100, and can correspond to the finger rest 14 of the nasal spray/spout nozzle 10. The upper exhaust airless-type spray/spout device 100 comprises a sliding bottom-lid body 160 capable of sliding upward in an interaction with the suction to exhaust an air having contained in the container 140. In the upper exhaust airless-type spray/spout device 100, an annular outer-peripheral surface part of the pump 120 (e.g., an upper annular outer-peripheral surface part 121 and a lower annular outer-peripheral surface part 122 of the pump), which can move in a seal state due to a pressure contact with an inner peripheral surface 141 of the container, is provided around the sliding bottom-lid body 160.

In other words, the upper exhaust airless-type spray/spout device comprises a container for storing a medicament, a pump capable of sucking the medicament from the container, and the nasal spray/spout nozzle for ejecting the medicament, wherein the container, the pump and the nasal spray/spout nozzle are in fluid communication in such order. The pump of the upper exhaust airless-type spray/spout device, which is capable of sucking the medicament from the container, is provided with a sliding bottom-lid body that is capable of sliding upward in an interaction with the suction, and thereby exhausting an inside air of the container.

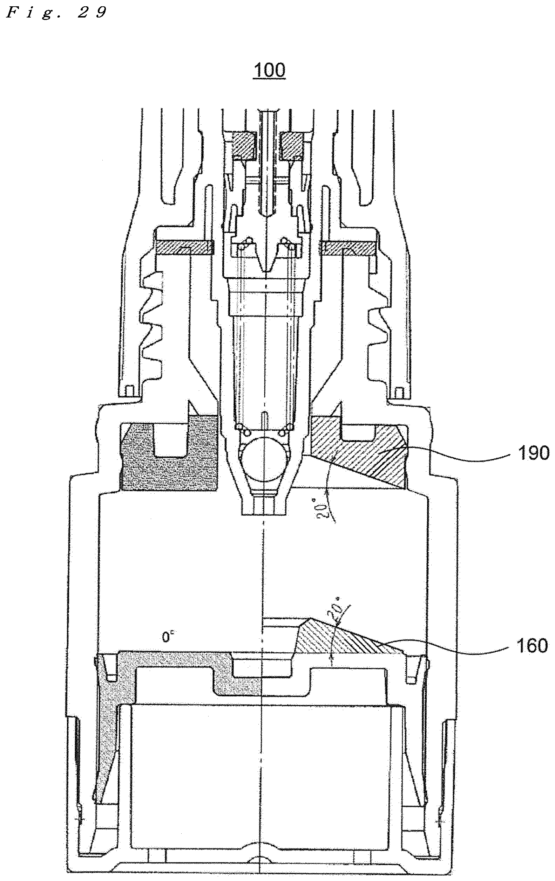

As shown in Fig. 29, the upper exhaust airless-type spray/spout device 100 is equipped with a ring 190 for excluding an unnecessary internal space, and also enables the sliding bottom-lid body 160 (which is usually flat) to take a predetermined angle. As such, the inside air of the upper exhaust airless-type spray/spout device 100 can be easily exhausted therefrom, and thereby making it possible to more greatly reduce the remaining amount of the medicament in the device at a point in time after the completion of using the device.

The upper exhaust airless-type spray/spout device as described above can ensure an accuracy of a spray or spout amount, the accuracy being normally required for the medicament as a medical product. When the predetermined angle of the sliding bottom-lid body is too smaller, an air space tends to remain at a shoulder of the body of the container upon charging with the medicament. When the predetermined angle of the sliding bottom-lid body is larger, an air space tends to remain at a skirt of a sliding bottom lid, which can be disadvantageous. Thus, the predetermined angle of the sliding bottom-lid body is preferably 5° or more and 30° or less, and more preferably 15° or more and 25° or less.

The upper exhaust airless-type spray/spout device does not involve taking into the outside air, and thereby is hardly contaminated by microorganisms attributed to the outside air. This means that the upper exhaust airless-type spray/spout device can be a significantly useful device especially in terms of using a pharmaceutical medicament. The pharmaceutical medicament for the upper exhaust airless-type spray/spout device does not require an antiseptic agent or a preservative more than necessary. Accordingly, the upper exhaust airless-type spray/spout device can be provided as a nasal administration system that can be significantly excellent in terms of its safety and manufacturing costs.

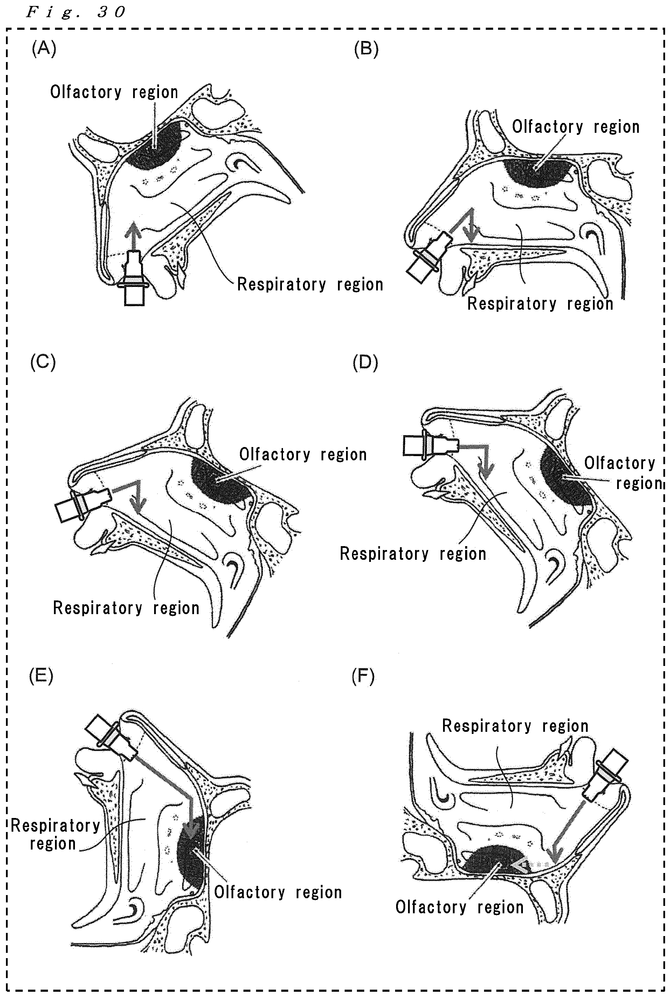

The upper exhaust airless-type spray/spout device according to the present invention enables the spray and/or spout with any administration angle, which can contribute to a more suitable utilization of the nasal spray/spout nozzle (see Figs. 30A to 30F). That is, an administration angle of the spray/spout device can be any angle selected from the range of 0° to 360°, or the spray/spout device can be suitably used within such angle range. For example, in a case of the upper exhaust airless-type spray/spout device for nasal use, a spray-type nasal medicament can be suitably used by variously changing the administration angle formed between an administration position (an angle of the head) and a spray container. That is, it is possible to spray and/or spout the medicament with an angle of around 0° to up to 45°, and also 45° or more and 90° or less, and further up to 180°.

When the head is tilted backward and the administration angle regarding the container of the device is 65° or more and 180° or less, an orientation of a nasal turbinate (or a nasal meatus) can be allowed to take from the horizontal one to the vertical one, which will enable the medicament to be first dispersed and suitably adhered onto a tip area of the nasal turbinate. In this regard, the medicament captured and deposited on the turbinate can be carried backward due to its flow state by gravity and also due to a ciliary motility of ciliated cells covering the mucous membrane, which will make it possible for the medicament to spread over a wider area of the nasal turbinate.

Accordingly, the administration angle between the administration position and the container of the upper exhaust airless-type spray/spout device may be any angle of 0° or more and 180° or less, preferably 45° or more and 180° or less, more preferably 65° or more and 180° or less, and the most preferably around 135°, which can be the most advantageous and desirable angle in that the nasal turbinate (or the nasal meatus) can be in the vertical orientation and the medicament can more suitably spread over the wider area of the nasal turbinate. The posture during the administration by use of the upper exhaust airless-type spray/spout device may be any one selected from a standing posture, a sitting position, a supine position, a recumbent position and the like, which means that the nasal medicament according to the present invention can be suitably used with any administration angle.

### (Syringe-type Spray/Spout Device)

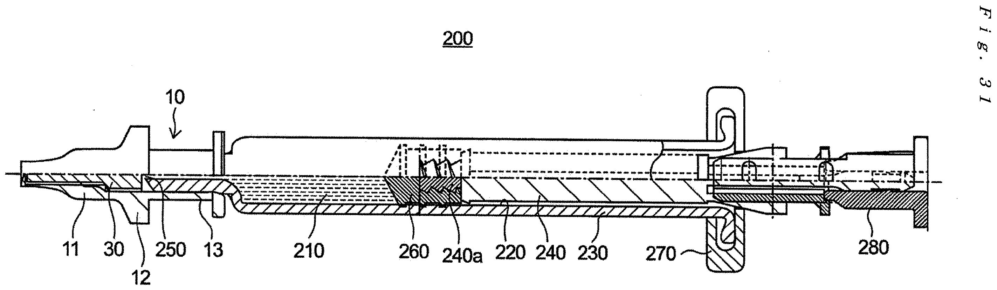

Fig. 31 illustrates a syringe-type spray/spout device 200 provided with the nasal spray/spout nozzle 10 according to one embodiment of the present disclosure. The syringe-type spray/spout device 200 comprises a syringe body 230 having a syringe barrel 220, a plunger rod 240 provided within the syringe barrel 220 of the syringe body 230, a piston 260 attached to the plunger rod 240 via a fixing portion 240a (the fixing portion being provided at a distal end of the plunger rod 240), and a finger rest 270 provided on the syringe body 230 (around a proximal end of the syringe body 230). More specifically, as illustrated in Fig. 31, the syringe-type spray/spout device 200 comprises the syringe body 230 (made of a synthetic resin or a glass) having the syringe barrel 220 capable of being charged with a medicament 210, the plunger rod 240 disposed within the syringe barrel 220, the piston 260 attached to the plunger rod 240 via the fixing portion 240a provided at the distal end of the plunger rod 240, the piston being configured to slide within the syringe barrel 220 to eject the medicament contained in the syringe barrel 220 from a tip opening 250 of the syringe body 230 (the tip opening being at a distal side of the syringe body 230), the finger rest 270, which is arranged on the the syringe body 230, for contributing to transmitting the force applied by a finger of a user (e.g., a doctor) to the plunger rod 240 (the finger rest being arranged on the syringe body 230 around the proximal end thereof), and a plunger operating portion 280.

As shown in Fig. 31, the syringe-type spray/spout device 200 comprises the nasal spray/spout nozzle 10 that is disposed such that the nozzle faces the tip opening 250 of the syringe body 230. In this regard, the nasal spray/spout nozzle 10 may be one as illustrated in Fig. 4. Such nasal spray/spout nozzle 10 may further include a protective cap (not shown) for protecting the tip portion 11 against contaminants and also protecting an undesirable impact on the tip portion 11. The protective cap may be configured to cover the whole of the nozzle. Alternatively, the protective cap may be configured to cover only the tip portion of the nozzle.

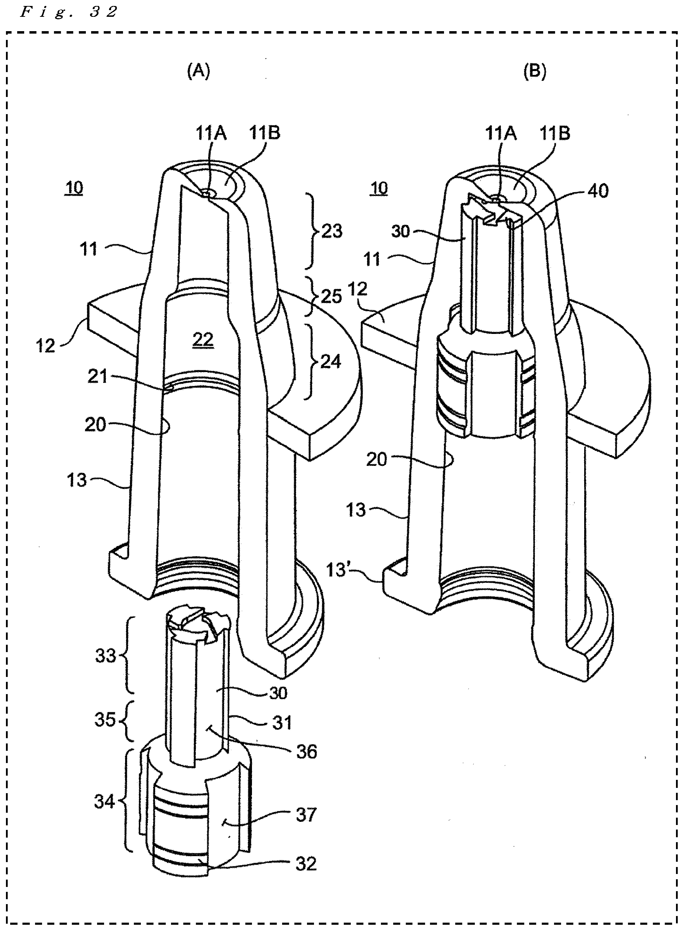

Figs. 32A and 32B are partially broken exploded perspective views illustrating a schematic configuration/structure of the nasal spray/spout nozzle 10 according to one embodiment of the present disclosure. As illustrated in Figs. 32A and 32B, the nasal spray/spout nozzle 10 can be provided as having the nozzle body 13 which is hollow and has a nozzle end face 11B provided with its nozzle exit port 11A, and a rod 30 which is solid and disposed within the hollow nozzle body as a filling rod. Fig. 32A illustrates a state at a point in time before the filling rod 30 is disposed/inserted into the body 13 of the nozzle. While on the other hand, Fig. 32B illustrates a state at a point in time after the filling rod 30 is disposed/inserted into the body 13 of the nozzle. The nozzle end face 11B as a nozzle exit face in the nozzle body 13 has approximately a circular shape, and the nozzle exit port 11A is positioned at the center of the nozzle end face 11B.

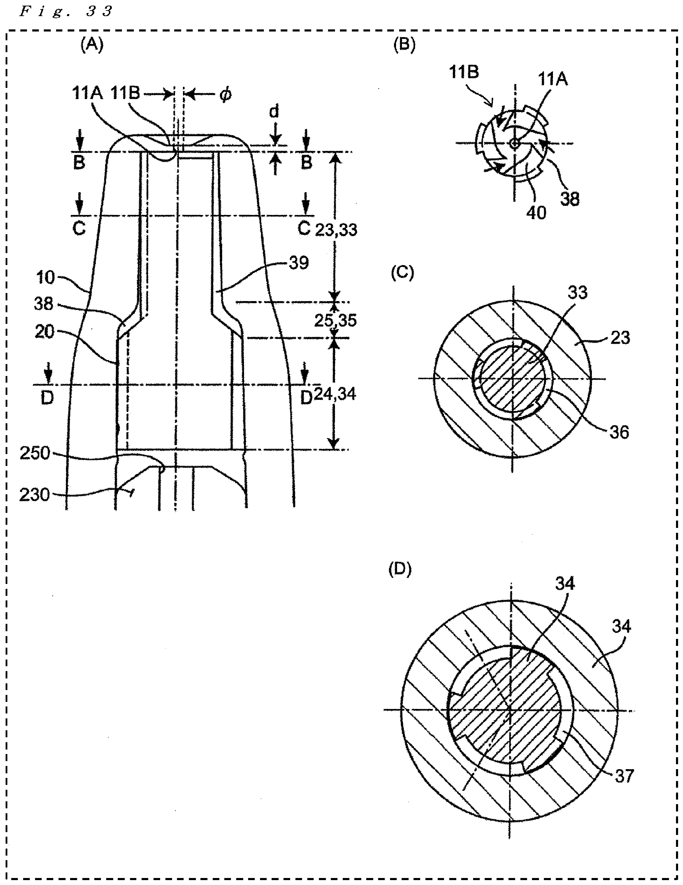

Fig. 33A is a vertical cross-sectional view of the nasal spray/spout nozzle 10 of Fig. 32B, as taken along a vertical plane passing through the nozzle exit port 11A. Figs. 33B to 33D are horizontal cross-sectional views as taken along a line B-B, a line C-C and a line D-D in Fig. 33A, respectively. An inner wall 20 of the hollow nozzle body 13 defines a hollow space 22 as an interior space of the body, and such hollow space 22 has approximately a cylindrical shape. As shown in Figs. 33C and 33D, the hollow space 22 has a smaller-diameter nozzle portion 23, a larger-diameter nozzle portion 24, and a nozzle shoulder 25. The smaller-diameter nozzle portion 23 is located closer to the nozzle exit port 11A of the nozzle body 13. The larger-diameter nozzle portion 24 is a larger portion in which a larger-diameter rod portion 34 of the filling rod 30 is positioned. The nozzle shoulder 25 is provided in such a manner that a diameter of the hollow space 22 reduces continuously or stepwise from the larger-diameter nozzle portion 24 to the smaller-diameter nozzle portion 23.

The solid rod as the filling rod 30, which is disposed/inserted within the nozzle body 13, has an outer wall 31 whose outer shape is substantially complementary to the inner wall 20 of the nozzle body 13 (i.e., the hollow space 22). The filling rod 30 has a smaller-diameter rod portion 33, the larger-diameter rod portion 34, and a rod shoulder 35 as illustrated in Figs. 32A, 33C and 33D. The rod shoulder 35 is provided in such a manner that a diameter of the rod reduces continuously or stepwise from the larger-diameter rod portion 34 to the smaller-diameter rod portion 33.

As illustrated in Fig. 32A, it is preferred that the inner wall 20 of the nozzle body 13 is provided with a raised portion 21, and the outer wall 31 of the rod 30 is provided with a recess 32 for receiving the raised portion 21. It is preferred in this case that the raised portion 21 and the recess 32 fit each other to be reliably secured when the rod 30 is disposed/inserted in the hollow space 22 of the nozzle body 13.

As can be clear from Figs. 32A, 32B and 33A to 33D, the rod 30 has a plurality of grooves 36 and grooves 37. The plurality of grooves 36 are spaced away from each other in a circumferential direction of the smaller-diameter rod portion 33, and the plurality of grooves 37 are spaced away from each other in a circumferential direction of the larger-diameter rod portion 34. The rod 30 can be disposed/inserted into the nozzle body 13 such that a gap 38 (Fig. 33A) between the nozzle shoulder 25 and the rod shoulder 35 is formed. As a result, the nasal spray/spout nozzle 10, which can be in an assembled state as in Fig. 32B, can be provided with a nozzle chamber 39 for allowing a fluid communication between the grooves 36, the grooves 37 and the gap 38 to guide the medicament 210 having delivered from the tip opening 250 of the syringe body 230 to the nozzle end face 11B of the nasal spray/spout nozzle 10 via the nozzle chamber 39.

Further, as shown in Fig. 33B, the rod 30 has a vortex-flow generator 40 that faces the nozzle end face 11B of the nasal spray/spout nozzle 10. The vortex-flow generator 40 is configured for generating a vortex flow of the medicament at a point in time before the medicament 210 having flown therein from respective grooves 36 of the smaller-diameter rod portion 33 is ejected from the nozzle exit port 11A of the nozzle body 13. More specifically, the vortex-flow generator 40, which constitutes the end of the smaller-diameter rod portion 33, extends in such a manner that it is displaced away from a vertical central axis passing the nozzle exit port 11A. This enables the flow of the medicament 210 to turn into the vortex flow at a point in time immediately before the medicament 210 is ejected from the nozzle exit port 11A. The vortex flow makes it possible for the ejection angle of the medicament to be widened, and thereby allowing the medicament to be sprayed and/or spouted more uniformly.

As can be clear from Figs. 33C and 33D, it is preferred that each of the grooves 36 of the smaller-diameter rod portion 33 is smaller/narrower than each of the grooves 37 of the larger-diameter rod portion 34, and thereby making it possible for a pressure of the medicament in the vortex-flow generator 40 to be increased at a point in time before being ejected from the nozzle exit port 11A. Since the rod is designed such that the diameter thereof reduces continuously or stepwise from the larger-diameter rod portion 34 to the smaller-diameter rod portion 33, a suitably deep insertion of the nozzle into the nasal cavity of the user (e.g., a patient) can be facilitated, leading to the suitable spraying/spout of the medicament around the inferior turbinate of the user and onto a deeper nasal region thereof. It is preferred that the diameter of the smaller-diameter rod portion 33 is sufficiently smaller than the naris of the user in terms of not giving the user a sense of fear.

The syringe-type spray/spout device according to the present disclosure comprises a syringe body for storing a medicament, and the nasal spray/spout nozzle that is in fluid communication with the syringe body. The syringe body in the syringe-type spray/spout device comprises a plunger rod and piston for ejecting the medicament, and also a finger rest and a plunger-operating portion that cooperate with the plunger rod. The nasal spray/spout nozzle in the syringe-type spray/spout device further comprises the filling rod disposed within the nasal spray/spout nozzle, wherein the nozzle chamber is provided between the filling rod and the inner wall of the nasal spray/spout nozzle and is in fluid communication with the nozzle exit port of the nozzle.

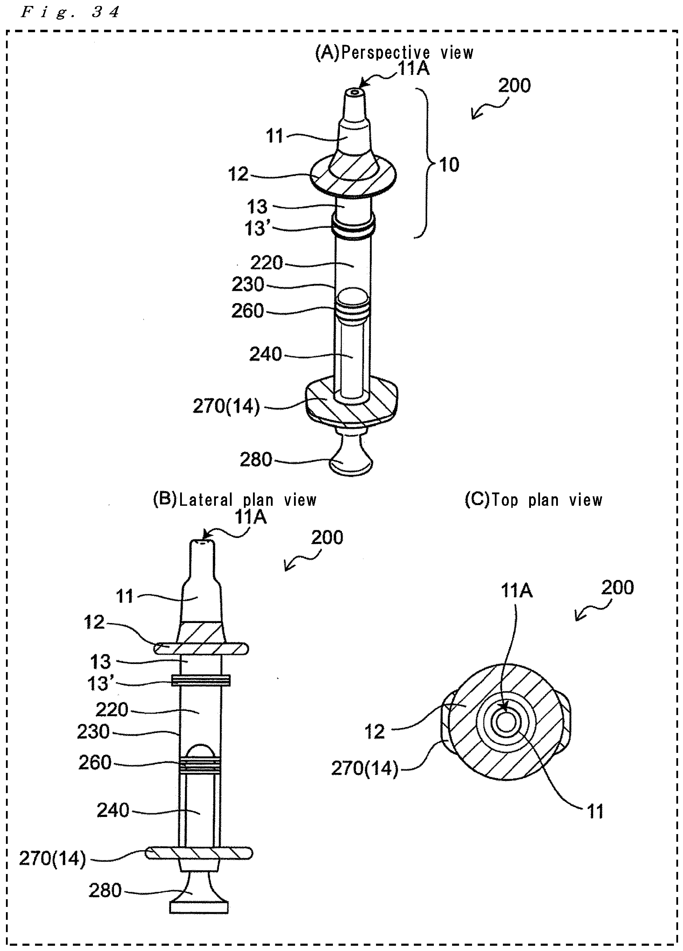

Fig. 34 illustrates the syringe-type spray/spout device 200 equipped with the nasal spray/spout nozzle 10 according to one embodiment of the present invention. In an embodiment shown in Fig. 34, the nasal spray/spout nozzle 10 has the nose rest 12, and the syringe body 230 has the finger rest 270.

For example, the outer contour of the nose rest 12 of the nozzle and the outer contour of the finger rest 14 of the syringe are each positioned to surround the outer contour of the tip portion 11 in a top plan view when viewed from the side of the nasal spray/spout nozzle 10. This configuration/form allows the suitable abutment such that a finger can be suitably acted in an act direction of the nasal spray/spout nozzle 10.

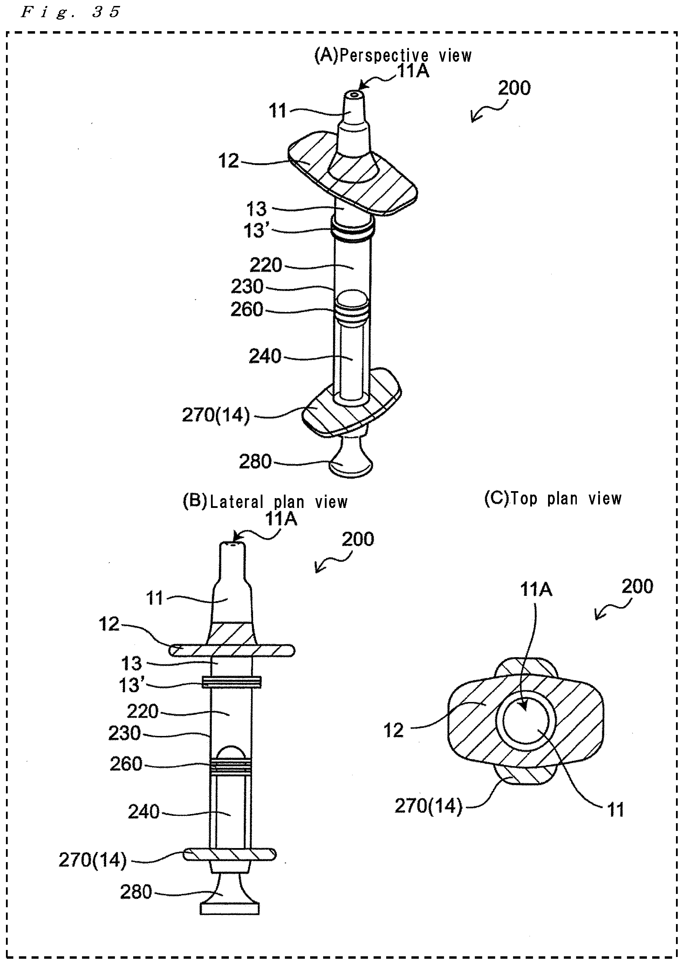

In one embodiment of the present disclosure, the nose rest 12 of the nasal spray/spout nozzle 10 and the finger rest 14 of the syringe body 230 may be each in a form of approximately an ellipse or approximately a rectangle (see Fig. 35), in which case the longest diameters of the nose rest 12 and the finger rest 14 are perpendicular to each other.

In the above embodiment, the longest diameter of the nose rest 12 of the nozzle and the longest diameter of the finger rest 14 of the syringe body may be approximately perpendicular to each other in a top plan view when viewed from the side of the nasal spray/spout nozzle 10. Specifically, the nose rest 12 and the finger rest 14 may each have approximately an elliptic shape or approximately a rectangular shape, in which case the longest diameter of the nose rest and the longest diameter of the finger rest may be approximately perpendicular to each other.

In other words, the nose rest 12 and the finger rest 14 can form approximately a cross shape in a top plan view when viewed from the side of the nasal spray/spout nozzle 10. In this regard, the longest diameter of the nose rest 12 of the nozzle and the longest diameter of the finger rest 14 of the syringe body may be in approximately a cross form with each other so as to be line-symmetric.

In other words, in a top plan view when viewed from the side of the nasal spray/spout nozzle 10, the nose rest 12 and the finger rest 14 have an overlap portion with each other, while an opposing direction of the two non-overlapping portions of the nose rest 12 and an opposing direction of the two non-overlapping portions of the finger rest 14 may be approximately perpendicular to each other.

Such configuration/form can facilitate positioning the nasal spray/spout nozzle with respect to the external naris more easily and more accurately. That is, it can be made particularly easier to suitably ensure the orientation of the nozzle exit port 11A within the nasal cavity.

### (Rest Article of the Present Disclosure)

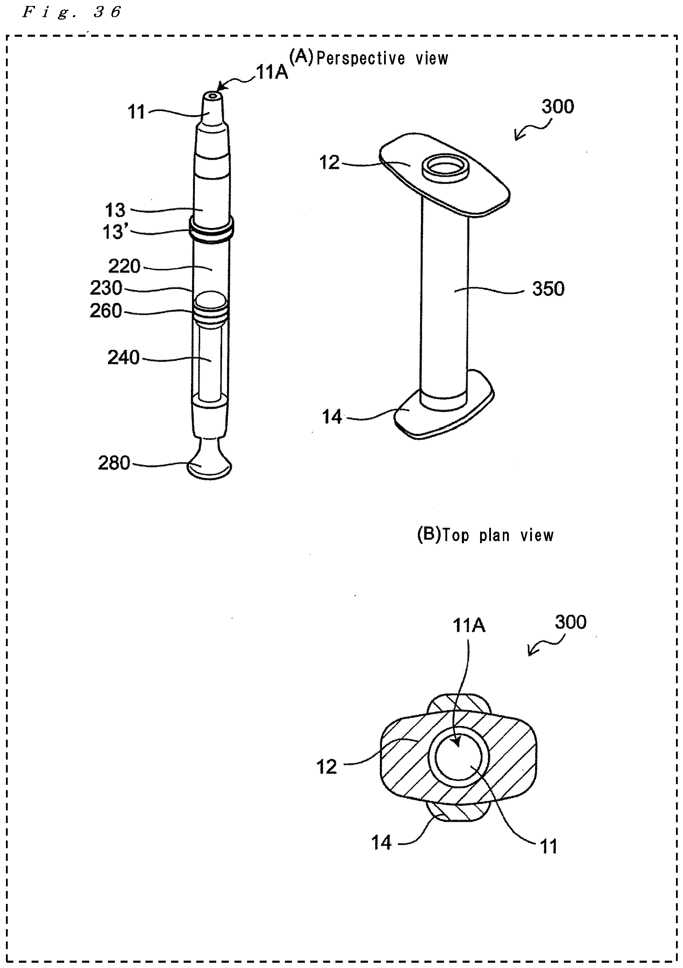

According to the technical idea of the present disclosure, there can be provided a nasal rest article 300 for nasal administration as shown in Fig. 36, for example. The rest article 300 comprises the nose rest 12 capable of abutting on the nose region around the external naris, and the finger rest 14. Such rest article 300 can be attached/mounted onto, for example, the conventional spray/spout device, the upper exhaust airless-type spray/spout device, or the syringe-type spray/spout device (hereinafter, also collectively referred to as a *"spray*/*spout device"*)*.* That is, the rest article 300 can correspond to a part product for the spray/spout device such that the rest article can provide the spray/spout device with the nose rest 12 and the finger rest 14.

As shown in Fig. 36, the nose rest 12 and the finger rest 14 are in an integration with each other in the rest article 300. For example, the nose rest 12 and the finger rest 14 are in connection to each other via a cylindrical part 350. With the spray/spout device having such rest article 300 (preferably by mounting/attaching the rest article 300 onto the spray/spout device from the side of the nozzle exit port of the device), the spray/spout device can be equipped with both the nose rest 12 and the finger rest 14. For example, the rest article 300 can be disposed such that a barrel portion of the upper exhaust airless-type spray/spout device or syringe-type spray/spout device is positioned inside the cylindrical part 350 of the rest article 300. With such rest article 300, the spray/spout device can be suitably provided with the nose rest 12 and the finger rest 14.

In the rest article 300 according to the present disclosure, the nose rest 12 and the finger rest 14 may each have approximately an elliptic shape or approximately a rectangular shape, in which case the longest diameter of the nose rest 12 and the longest diameter of the finger rest 14 may be perpendicular to each other (see Fig. 36). That is, the nose rest 12 and the finger rest 14 in the rest article 300 may form approximately a cross shape in a top plan view of the rest article 300. While the nose rest 12 and the finger rest 14 have an overlap portion with each other in a top plan view of the rest article 300, an opposing direction of the two non-overlapping portions regarding the nose rest 12 and an opposing direction of the two non-overlapping portions regarding the finger rest 14 may be approximately perpendicular to each other. Use of such a nozzle article can facilitate positioning the nozzle with respect to the external naris more easily and more accurately. As illustrated in Fig. 36, the spaced distance between the nose rest 12 and the finger rest 14 in the rest article 300 may be, for example, in the range of about 50 mm to about 80 mm to be more suitably used for the syringe-type spray/spout device.

### (Characteristics of Medicament)

The properties (e.g., pH, viscosity, surface tension, osmotic pressure, and the like) of the medicament to be used for the spray/spout device are not particularly restricted. As for the viscosity of the medicament, it can affect spray/spout of the nozzle, but such viscosity is not particularly restricted for use in the present disclosure. For example, the medicament may have a form of a liquid, gel or slurry. For example, the viscosity of the medicament may be 1 mPa·s or more and 5000 Pa·s or less. In one embodiment, the viscosity of the medicament is 250 Pa·s or more and 2500 Pa·s or less.

In a case where the viscosity of the medicament is 250 Pa·s or less, there can be a tendency that a loss of the once-delivered medicament from the target region is caused by a liquid dripping phenomenon even if the medicament reaches the target region. While on the other hand, in a case where the viscosity is 5000 Pa·s or more, a predetermined distance "C" 51 from the nozzle exit port, which can have such a state that the medicament forms its film from the nozzle exit port (i.e., a narrower-diffusion region), becomes longer, and thus the film is less likely to be suitably broken. This can lead to the medication remaining in the target region.

### (Method of Using Spray/Spout Device)

As an example, Fig. 37 illustrates a method of using the upper exhaust airless-type spray/spout device 100 equipped with the nasal spray/spout nozzle 10 of Fig. 7. The nasal spray/spout nozzle 10 shown in Fig. 37 has the nose rest 12 and the finger rest 14.

The user can use the spray/spout device 100 in accordance with the following procedures, for example.
(1) Two fingers (e.g., the index finger and the middle finger) are disposed onto the finger rest 14 such that they are faced with each other across nozzle body 13.
(2) The nasal spray/spout nozzle 10 is oriented such that the disposed two fingers can form approximately a parallel relationship with the longitudinal direction of the nasal columella.
(3) The tip portion 11 of the nasal spray/spout nozzle 10 is inserted into the nasal cavity. In this regard, the tip portion 11 is inserted until the nose rest 12 abuts against the nose region located around the external naris (for example, against the nasal columella, the nasal ala, and the like). See Figs. 37 and 38.
(4) By allowing the finger rest 14 to act toward the distal side of the nasal spray/spout nozzle 10 (i.e., in the direction of the act in the drawing), the medicament can be ejected from the nozzle into the nasal cavity.

In the nasal spray/spout nozzle 10 shown in Fig. 37, the longest diameter N-N' of the nose rest 12 and the longest diameter F-F' of the finger rest 14 may be approximately perpendicular to each other (see Fig. 7C). When such nasal spray/spout nozzle 10 is disposed and oriented as in the above procedure (2), the longest-diameter direction of the nose rest 12 can be facilitated to naturally approximately be aligned with the direction connecting the nasal columella and the nasal ala (see Fig. 38). As a result, it can be made possible for the nose rest 12 to more suitably fit the nasal columella and the nasal ala.

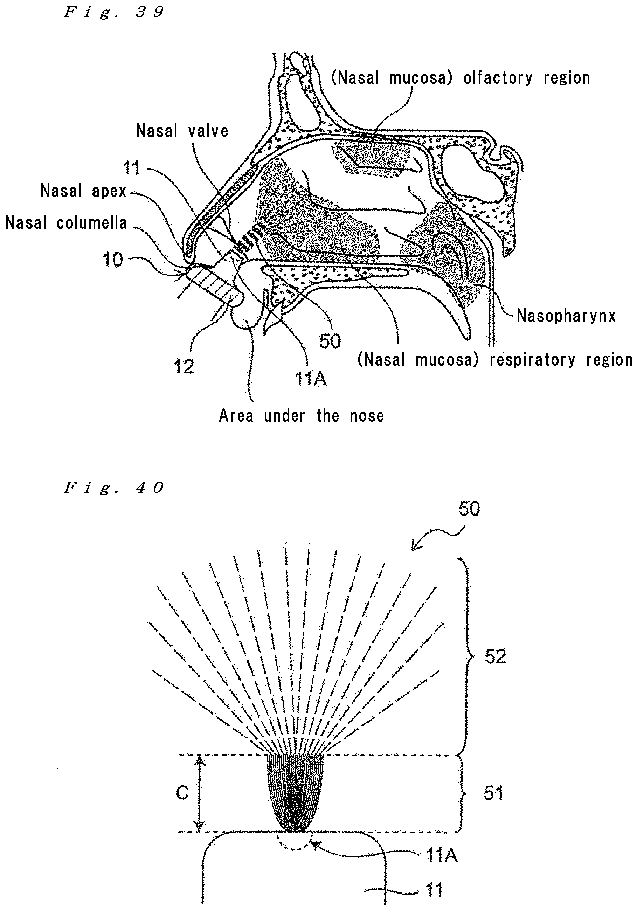

Fig. 39 shows one embodiment of the nasal spray/spout nozzle 10 having inserted into the nasal cavity in accordance with the above procedures.

As shown in Fig. 39, the nose rest 12 is allowed to abut onto the nose region located around the external naris, and thereby making it possible to more easily ensure the inserted position and orientation of the nozzle exit port 11A in such a manner that the tip portion 11 or the ejected medicament 50 can suitably divert a narrower nasal cavity located around the nasal valve. Accordingly, the medicament delivery rate onto the target region is likely to become higher, and/or the medicament is likely to be suitably delivered over a larger area regarding the target region (e.g., over the larger area of the respiratory region, the olfactory region, and/or the nasopharynx region).

The ejected medicament 50 includes a narrower-diffusion region 51 at a point in time immediately after the ejection, and a wider-diffusion region 52 at a point time when finer droplets as compared with those of the narrower-diffusion region 51 are formed (see Fig. 40). While not wishing to be bound by any theory, the medicament at a point in time immediately after the ejection accompanies its film state formed at an outer edge of the ejected medication, and is in a relatively converged state, but when the medicament goes through a certain ejection distance, the film can be broken so that the medicament becomes finer droplets. That is, breaking the film of the medicament can make it possible for the medicament to turn into finer droplets thereof at such a boundary position that an ejection distance "C" is 5 mm or more and 15 mm or less with respect to the nozzle exit port 11A.

In order to deliver the medicament over the larger area of the target region, it may be important that the tip portion 11 of the nozzle or the narrower-diffusion region 51 of the ejected medicament 50 can divert the narrower nasal cavity located around the nasal valve (see Figs. 39 and 40). In this regard, the distance from the external naris to the nasal valve may be about 20 mm.

The length dimension of the tip portion 11 in the nasal spray/spout nozzle 10 according to the present disclosure, which is for example 5 mm or more and 30 mm or less, can allow the tip portion 11 of the nozzle or the narrower-diffusion region 51 of the medicament 50 to suitably divert the narrower nasal cavity.

In terms of preventing an undesirable contact of the nozzle with the inner wall of the nasal cavity located around the nasal valve and also in terms of suitably delivering the medicament to the larger area of the target region, it is preferred that the narrower-diffusion region 51 of the ejected medicament 50 suitably diverts the narrower nasal cavity located around the nasal valve (see Figs. 39 and 40).

To this end, the length dimension of the tip portion 11 may be, for example, 10 mm or more and 25 mm or less, 7 mm or more and 18 mm or less, 8 mm or more and 17 mm or less, 9 mm or more and 16 mm or less, or 10 mm or more and 15 mm or less (e.g., about 15 mm just as one example). As used herein, the length dimension of the tip portion 11 may refer to a length from the proximal end of the nose rest 12 to the nozzle face having the nozzle exit port 11A.

In particular, such length dimension of the tip portion 11 may facilitate positioning the nozzle exit port 11A immediately around the nasal valve, and thereby making it possible for the narrower-diffusion region 51 of the ejected medicament 50 to more easily divert the narrower nasal cavity located around the nasal valve. With such length dimension of the tip portion of the nozzle, the distance from the nozzle exit port 11A to the target region can be suitably ensured, which will lead to the more effective delivery of the medicament over the larger area of the target region.

Although some embodiments of the present disclosure have been hereinbefore described, they are regarded for illustrative purpose regarding the typical ones. The present disclosure is not limited to these embodiments. It would be readily appreciated by those skilled in the art that various embodiments are possible.

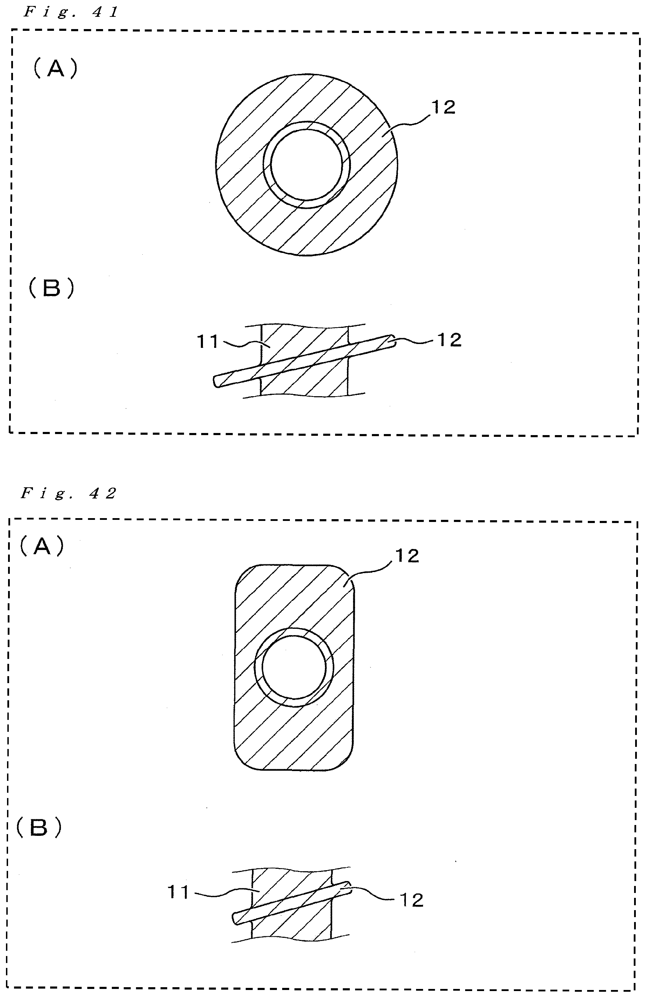

For example, the nose rest 12 having a difference in thickness has been described with reference to Figs. 12 and 13. As described above, the nose rest 12 with such thickness difference has a partially or wholly inclined surface as an upper surface of the nose rest (e.g., as a surface at the proximal side of the nozzle). The inclined form of the nose rest can be embodied in various manner. For example, the face of the nose rest 12 may be inclined as shown in each of Figs. 41 and 42. That is, in a lateral plan view as shown in each of Figs. 41B and 42B, the nose rest 12 having a constant thickness (substantially a constant thickness) may be inclined such that it forms an angle with respect to a direction perpendicular to the axis of the tip portion 11.

Further, while the term *"target region"*has been used in the above description, the present invention is not necessarily limited to this term. The target region is substantially synonymous with a "target site". That is, the present invention may be understood together with the expression of the "target site" instead of or in addition to the *"target region".* When the rhinitis or the like is assumed, the target site may be a nasal turbinate (inferior turbinate and/or middle turbinate). For example, in a case where the turbinate is swollen due to the allergic rhinitis or the like, and thereby causing a clogged state of the nose, the use of the nasal spray/spout nozzle according to the present invention can make it possible to increase the delivery rate of the medicament onto the inferior turbinate and/or the middle turbinate. In a case of using a nasal formulation that is expected to have a systemic effect by nasal mucosa absorption through a wide nasal mucosa of the respiratory region of the nasal cavity, the target site may be a nasal cavity intermediate site. In a case of using the nasal formulation that is expected to give an antibody production effect by a nasal vaccine due to a strong immune response of the nasopharynx region, the target site may be a nasopharyngeal site. Further, there can be a route directly leading to the brain from an olfactory mucosa, and thus an increase in the amount of the medicament having delivered onto the olfactory mucosa can result in an increase in the possibility of a suitable delivery of the medicament from the nose to the brain. Accordingly, in a case of using the nasal formulation that is expected to have a suitable effect on the central nervous system, the target site may be the olfactory mucosa.

Furthermore, in the above description, an outer structure/form of the nasal spray/spout nozzle has been mainly described. The present disclosure has no particular limitation in terms of an interior structure/form of the nasal spray/spout nozzle, or an inside structure/form of the nasal spray/spout nozzle. The interior structure/form of the nasal spray/spout nozzle and the inside structure/form of the nasal spray/spout nozzle according to the present disclosure may be similar to those of a conventional nozzle that is used as a nasal spray/spout nozzle for nasal administration. That is, the interior structure/form of the nasal spray/spout nozzle and the inside structure/form of the nasal spray/spout nozzle are not particularly limited or restricted as long as they can contribute to a nozzle function (e.g., spray/spout function of the medicament). Any conventional interior or inside structure/form of the nozzle is applicable to the present disclosure.

### EXAMPLES

Examples and Comparative examples, which can be related to the present disclosure, will be described below.

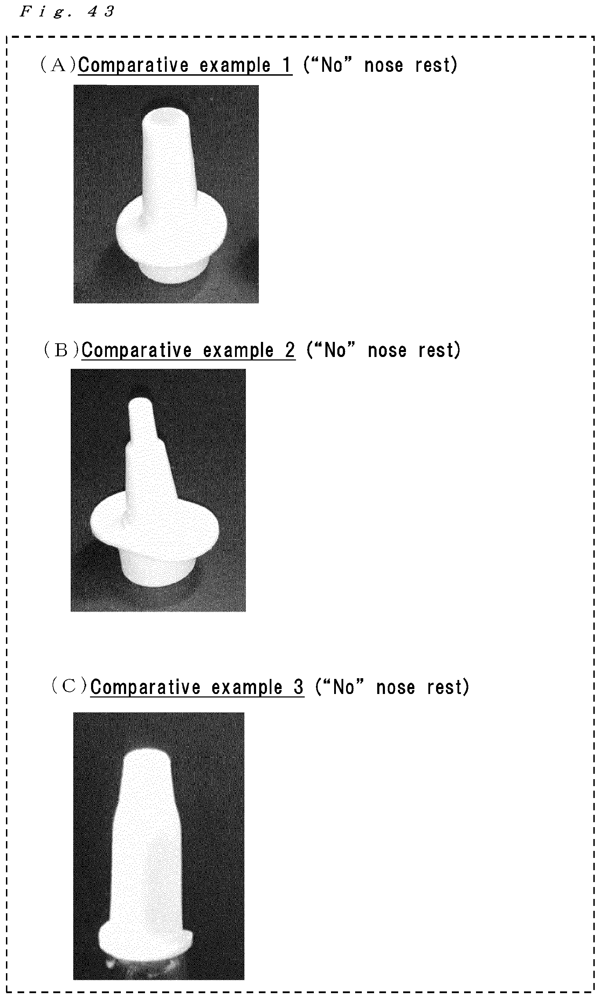

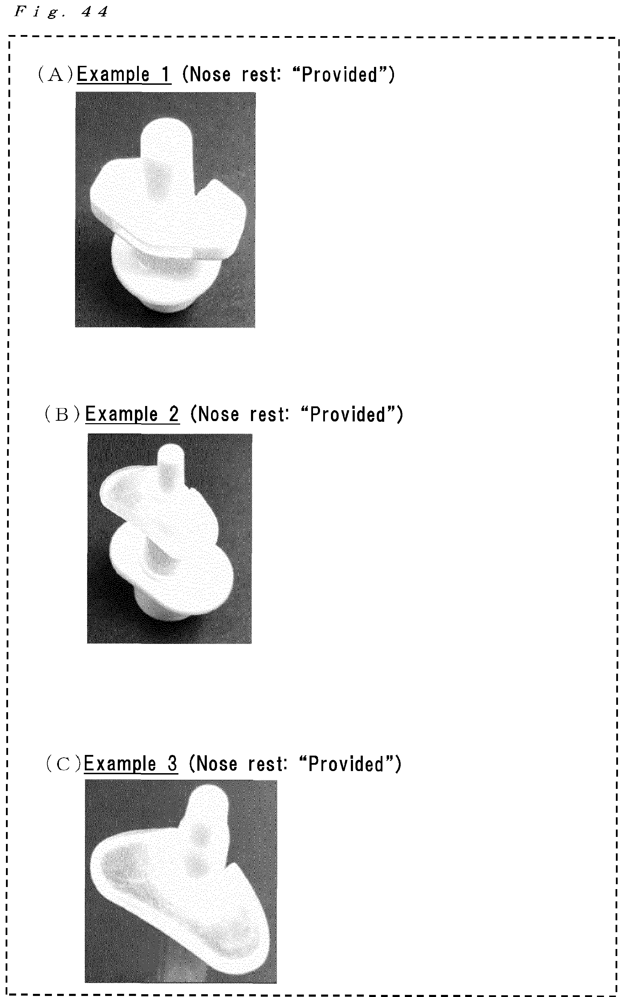

Demonstration tests were performed using the following test nozzles, each of which simulated a nasal spray/spout nozzle for nasal administration.
- Comparative example 1: A nasal spray/spout nozzle without a nose rest being provided. The nozzle of Comparative example 1 had no nose rest therein. See Fig. 43A.
   - Diameter dimension of an end face having a nozzle exit port: 8 mm
   - Spray amount: 100 mg (wherein the nozzle was used with being connected to a pump with a spray amount of 100 mg)
- Comparative example 2: A nasal spray/spout nozzle without a nose rest being provided. The nozzle of Comparative example 2 had no nose rest therein. See Fig. 43B.
   - Diameter dimension of an end face having a nozzle exit port: 4 mm
   - Spray amount: 100 mg (wherein the nozzle was used with being connected to a pump with a spray amount of 100 mg)
- Comparative example 3: A nasal spray/spout nozzle without a nose rest being provided. The nozzle of Comparative example 3 had no nose rest therein. See Fig. 43C.
   - Diameter dimension of an end face having a nozzle exit port: 4.5 mm
   - Spray amount: 250 mg (wherein the nozzle was used with being connected to a 1.0 mL syringe barrel)
- Example 1: A nasal spray/spout nozzle that was the same as that of Comparative example 1 except that a nose rest was provided in the nozzle of Example 1. The nozzle of Example 1 had the nose rest therein. See Fig. 44A.
   - The nose rest was provided in the nozzle such that the length dimension of a tip portion in the nozzle was 10 mm or 15 mm.
- Example 2: A nasal spray/spout nozzle that was the same as that of Comparative example 2 except that a nose rest was provided in the nozzle of Example 2. The nozzle of Example 2 had the nose rest therein. See Fig. 44B.
   - The nose rest was provided in the nozzle such that the length dimension of a tip portion in the nozzle was 15 mm.
- Example 3: A nasal spray/spout nozzle that was the same as that of Comparative example 3 except that a nose rest was provided in the nozzle of Example 3. The nozzle of Example 3 had the nose rest therein. See Fig. 44C.
   - The nose rest was provided such that the length dimension of a tip portion in the nozzle was 10 mm, 15 mm or 20 mm.

### Confirmation of Spray Condition

A spray condition/state of a medicament upon use of the nasal spray/spout nozzle was confirmed. In this regard, a liquid medicament was prepared in accordance with a production method described in Patent JP-B2-5185109 of Toko Yakuhin Kogyo Co., Ltd. This was in consideration of a matter that determining a mass of the liquid medicament having delivered and deposited onto a target site of the test required a stronger adhesion of the medicament to the target site.

As a result, it was satisfactorily confirmed that the above nasal spray/spout nozzles had been all capable of sufficiently spraying the medicament with fine mist thereof regardless of an ejection angle and a medicament viscosity.

Specifically, it was confirmed that, regardless of the ejection angle and the medicament viscosity, the nasal spray/spout nozzles according to the above Comparative examples 1 to 3 and Examples 1 to 3 had all had the spray state whose droplet diameter distribution of 10 µm to 100 µm accounted for 70% or more.

### Confirmatory Test on Effect of Nose rest

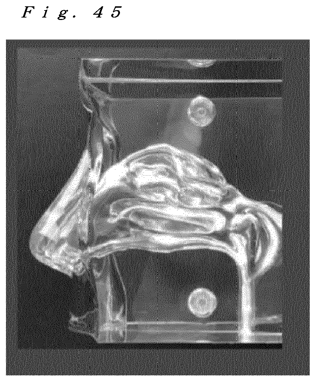

A three-dimensional model having a nasal cavity therein, which had been made of silicon, and manufactured by KOKEN CO., LTD, was used for the demonstration tests (see Fig. 45). Fig. 46 shows a position of the nozzle exit port and an angle of the nasal spray/spout nozzle during the test using the model. In Fig. 46A, *"angle α*"as an angle formed with respect to a plane parallel to the nasal septum is shown. In Fig. 46B, *"angle β*" as an angle formed with respect to a plane perpendicular to the nasal septum is shown.

In a case where a target site for which a spray was performed by the spray/spout nozzle was the *"nasal turbinate",* a screen was removably set in front of the inferior turbinate/the middle turbinate. Masses of the screen at a point in time before and after the spraying of the medicament by the nasal spray/spout nozzle were measured. Then, the deposited amount of the medicament on the screen was calculated from a difference in the measured masses, and also the deposited mass of the medicament on the nasal septum area located behind the screen was measured so as to finally determine the amount of the medicament having had delivered onto the nasal turbinate. In a case where the target site for which the spray was performed by the spray/spout nozzle was the *"nasal cavity intermediate site",* the deposited mass of the medicament on a rear section of the model (such section having had been prepared by cutting the model in the vertical direction at the center of the nasal cavity of the model) was measured so as to determine the amount of the medicament having had delivered onto the nasal cavity intermediate site. In a case where the target site for which the spray was performed by the spray/spout nozzle was the *"nasopharynx site",* the deposited mass of the medicament on a rear section of the model (such section having had been prepared by cutting the model in the vertical direction at the nasopharynx site corresponding to a rear part/face of the model) was measured so as to determine the amount of the medicament having had delivered onto the nasopharynx site. In a case where the target site for which the spray was performed by the spray/spout nozzle was the *"olfactory mucosa site",* the deposited mass of the medicament on an upper section of the model (such section having had been prepared by cutting the model in the horizontal direction) was measured so as to determine the amount of the medicament having had delivered onto the olfactory mucosa site.

*A "delivery rate on target site"* was calculated from a ratio of the amount of the medicament having had delivered onto the target site to the spray amount of the nozzle. The tests were performed a plurality of times to give variations in the *"delivery rate on target site"*by a relative standard deviation (i.e., *"RSD"*).

The results are shown below in Tables 1 to 16.

**[Table 1]**

| | Number of tests [Time] | Spray angle [° ] | Nozzle angle *α* [° ] | Nozzle angle *β* [° ] | Inserted length of nozzle [mm] | Target site | Delivery rate on target site [%] | RSD [%] | Change in delivery rate |
|---|---|---|---|---|---|---|---|---|---|
| Comparative example 1 - 1 | 10 | 62 | 30 | 5 | 10 | Nasal turbinate | 26.5 | 33.2 | - |
| Example 1 - 1 | 10 | 62 | 30 | 5 | 10 | Nasal turbinate | 39.4 | 11.1 | 49% Up |

**[Table 2]**

| | Number of tests [Time] | Spray angle [° ] | Nozzle angle *α* [° ] | Nozzle angle*β* [° ] | Inserted length of nozzle [mm] | Target site | Delivery rate on target site [%] | RSD [%] | Change in delivery rate |
|---|---|---|---|---|---|---|---|---|---|
| Comparative example 1-2 | 10 | 49 | 30 | 5 | 10 | Nasal turbinate | 46.4 | 33.0 | - |
| Example 1-2 | 10 | 49 | 30 | 5 | 10 | Nasal turbinate | 61.5 | 8.1 | 33% Up |

**[Table 3]**

| | Number of tests [Time] | Spray angle [° ] | Nozzle angle *α* [° ] | Nozzle angle *β* [° ] | Inserted length of nozzle [mm] | Target site | Delivery rate on target site [%] | RSD [%] | Change in delivery rate |
|---|---|---|---|---|---|---|---|---|---|
| Comparative example 1 - 3 | 3 | 66 | 30 | 5 | 10 | Nasal turbinate | 42.1 | 24.9 | - |
| Example 1 - 3 | 3 | 66 | 30 | 5 | 10 | Nasal turbinate | 55.0 | 2.2 | 31% Up |

**[Table 4]**

| | Number of tests [Time] | Spray angle [° ] | Nozzle angle *α* [° ] | Nozzle angle*β* [° ] | Inserted length of nozzle [mm] | Target site | Delivery rate on target site [%] | RSD [%] | Change in delivery rate |
|---|---|---|---|---|---|---|---|---|---|
| Comparative example 1-4 | 3 | 66 | 15 | 5 | 10 | Nasal turbinate | 55.7 | 11.4 | - |
| Example 1-4 | 3 | 66 | 15 | 5 | 10 | Nasal turbinate | 68.0 | 2.1 | 22% Up |

**[Table 5]**

| | Number of tests [Time] | Spray angle [° ] | Nozzle angle *α* [° ] | Nozzle angle *β* [° ] | Inserted length of nozzle [mm] | Target site | Delivery rate on target site [%] | RSD [%] | Change in delivery rate |
|---|---|---|---|---|---|---|---|---|---|
| Comparative example 1-5 | 3 | 66 | 30 | 5 | 15 | Nasal turbinate | 56.2 | 11.2 | - |
| Example 1-5 | 3 | 66 | 30 | 5 | 15 | Nasal turbinate | 83.3 | 3.8 | 48% Up |

**[Table 6]**

| | Number of tests [Time] | Spray angle [° ] | Nozzle angle *α* [° ] | Nozzle angle *β* [° ] | Inserted length of nozzle [mm] | Target site | Delivery rate on target site [%] | RSD [%] | Change in delivery rate |
|---|---|---|---|---|---|---|---|---|---|
| Comparative example 1 - 6 | 3 | 49 | 30 | 5 | 10 | Nasal cavity intermediate site | 9.1 | 29.8 | - |
| Example 1 - 6 | 3 | 49 | 30 | 5 | 10 | Nasal cavity intermediate site | 11.9 | 10.2 | 31% Up |

**[Table 7]**

| | Number of tests [Time] | Spray angle [° ] | Nozzle angle *α* [° ] | Nozzle angle *β* [° ] | Inserted length of nozzle [mm] | Target site | Delivery rate on target site [%] | RSD [%] | Change in delivery rate |
|---|---|---|---|---|---|---|---|---|---|
| Comparative example 1 - 7 | 3 | 49 | 15 | 5 | 15 | Nasal cavity intermediate site | 27.4 | 35.2 | - |
| Example 1-7 | 3 | 49 | 15 | 5 | 15 | Nasal cavity intermediate site | 34.5 | 24.3 | 26% Up |

**[Table 8]**

| | Number of tests [Time] | Spray angle [° ] | Nozzle angle *α* [° ] | Nozzle angle *β* [° ] | Inserted length of nozzle [mm] | Target site | Delivery rate on target site [%] | RSD [%] | Change in delivery rate |
|---|---|---|---|---|---|---|---|---|---|
| Comparative example 1-8 | 3 | 35 | 0 | 0 | 15 | Nasopharynx site | 8.6 | 29.2 | - |
| Example 1-8 | 3 | 35 | 0 | 0 | 15 | Nasopharynx site | 10.7 | 9.8 | 24% Up |

**[Table 9]**

| | Number of tests [Time] | Spray angle [° ] | Nozzle angle *α* [° ] | Nozzle angle *β* [° ] | Inserted length of nozzle [mm] | Target site | Delivery rate on target site [%] | RSD [%] | Change in delivery rate |
|---|---|---|---|---|---|---|---|---|---|
| Comparative example 2-1 | 3 | 35 | 60 | 0 | 15 | Olfactory mucosa site | 3.6 | 28.2 | - |
| Example 2-1 | 3 | 35 | 60 | 0 | 15 | Olfactory mucosa site | 8.2 | 18.8 | 128% Up |

**[Table 10]**

| | Number of tests [Time] | Spray angle [° ] | Nozzle angle *α* [° ] | Nozzle angle *β* [° ] | Inserted length of nozzle [mm] | Target site | Delivery rate on target site [%] | RSD [%] | Change in delivery rate |
|---|---|---|---|---|---|---|---|---|---|
| Comparative example 2-2 | 3 | 26 | 60 | 0 | 15 | Olfactory mucosa site | 10.9 | 9.1 | - |
| Example 2-2 | 3 | 26 | 60 | 0 | 15 | Olfactory mucosa site | 12.7 | 6.3 | 17% Up |

**[Table 11]**

| | Number of tests [Time] | Spray angle [° ] | Nozzle angle *α* [° ] | Nozzle angle *β* [° ] | Inserted length of nozzle [mm] | Target site | Delivery rate on target site [%] | RSD [%] | Change in delivery rate |
|---|---|---|---|---|---|---|---|---|---|
| Comparative example 3-1 | 10 | 63 | 30 | 5 | 15 | Nasal turbinate | 41.8 | 27.4 | - |
| Example 3-1 | 10 | 63 | 30 | 5 | 15 | Nasal turbinate | 54.9 | 6.3 | 31% Up |

**[Table 12]**

| | Number of tests [Time] | Spray angle [° ] | Nozzle angle *α* [° ] | Nozzle angle*β* [° ] | Inserted length of nozzle [mm] | Target site | Delivery rate on target site [96] | RSD [%] | Change in delivery rate |
|---|---|---|---|---|---|---|---|---|---|
| Comparative example 3-2 | 3 | 66 | 15 | 5 | 20 | Nasal turbinate | 78.4 | 4.1 | - |
| Example 3-2 | 3 | 66 | 15 | 5 | 20 | Nasal turbinate | 89.4 | 3.1 | 14% Up |

**[Table 13]**

| | Number of tests [Time] | Spray angle [° ] | Nozzle angle *α* [° ] | Nozzle angle *β* [° ] | Inserted length of nozzle [mm] | Target site | Delivery rate on target site [%] | RSD [%] | Change in delivery rate |
|---|---|---|---|---|---|---|---|---|---|
| Comparative example 3-3 | 3 | 42 | 15 | 0 | 10 | Nasal cavity intermediate site | 54.7 | 13.5 | - |
| Example 3-3 | 3 | 42 | 15 | 0 | 10 | Nasal cavity intermediate site | 64.5 | 7.2 | 18% Up |

**[Table 14]**

| | Number of tests [Time] | Spray angle [° ] | Nozzle angle *α* [° ] | Nozzle angle *β* [° ] | Inserted length of nozzle [mm] | Target site | Delivery rate on target site [%] | RSD [%] | Change in delivery rate |
|---|---|---|---|---|---|---|---|---|---|
| Comparative example 3-4 | 3 | 42 | 15 | 0 | 15 | Nasal cavity intermediate site | 68.5 | 9.4 | - |
| Example 3-4 | 3 | 42 | 15 | 0 | 15 | Nasal cavity intermediate site | 75.7 | 6.3 | 11% Up |

**[Table 15]**

| | Number of tests [Time] | Spray angle [° ] | Nozzle angle *α* [° ] | Nozzle angle *β* [° ] | Inserted length of nozzle [mm] | Target site | Delivery rate on target site [%] | RSD [%] | Change in delivery rate |
|---|---|---|---|---|---|---|---|---|---|
| Comparative example 3-5 | 3 | 27 | 0 | 0 | 15 | Nasopharynx site | 20.5 | 32.3 | - |
| Example 3-5 | 3 | 27 | 0 | 0 | 15 | Nasopharynx site | 29.0 | 12.9 | 41% Up |

**[Table 16]**

| | Number of tests [Time] | Spray angle [° ] | Nozzle angle *α* [° ] | Nozzle angle *β* [° ] | Inserted length of nozzle [mm] | Target site | Delivery rate on target site [%] | RSD [%] | Change in delivery rate |
|---|---|---|---|---|---|---|---|---|---|
| Comparative example 3-6 | 3 | 27 | 60 | 0 | 15 | Olfactory mucosa site | 9.3 | 16.9 | - |
| Example 3-6 | 3 | 27 | 60 | 0 | 15 | Olfactory mucosa site | 12.0 | 11.9 | 29% Up |

As shown in the above Tables 1 to 16, it has been found out that the nasal spray/spout nozzles with the nose rest being provided in each of them (i.e., Examples 1 to 3) each have a higher medicament delivery rate with respect to each of the target sites than that of the nasal spray/spout nozzles with no nose rest being provided in each of them (i.e., Comparative examples 1 to 3). It has been also found out that the nasal spray/spout nozzles with the nose rest being provided in each of them (i.e., Examples 1 to 3) can each suppress variations in the medicament delivery rate more than those of the nasal spray/spout nozzles with no nose rest being provided in each of them (i.e., Comparative examples 1 to 3). Consequently, it has been confirmed that the nasal spray/spout nozzle according to the present disclosure can further improve the performance of the medicament delivery.

### INDUSTRIAL APPLICABILITY

The nasal spray/spout nozzle according to the present disclosure can have a more suitable delivery efficiency of the medicament with respect to the target region having the complex intranasal structure. More specifically, by allowing the nose rest of the nasal spray/spout nozzle according to the present invention to abut against the nose region located around the external naris, the inserted position of the nozzle exit port within the nasal cavity can be suitably ensured. This means that the present disclosure can suitably achieve a realization of a nasal spray/spout-type medical device capable of delivering a medicament over a wider range of nasal target regions with a higher medicament efficacy.

### DESCRIPTION OF REFERENCE NUMERALS

10: Spray/spout nozzle for nasal administration
11: Tip portion
   11A: Nozzle exit port
   11B: Nozzle end face (Nozzle exit end face)
12: Nose rest
   12E: Proximal end of nose rest
   12S: Inclined face/surface of nose rest
13: Nozzle body
   13': Protrusion for connecting with a syringe
14: Finger rest
   14': Finger guide
15: Directive marker for insertion or Fit surface
   15a: Notch surface/face
20: Inner wall of nozzle body
   21: Raised portion
   22: Internal hollow space
   23: Smaller-diameter nozzle portion
   24: Larger-diameter nozzle portion
   25: Nozzle shoulder
30: Filling rod (filling bar)
   31: Outer wall of filling rod
   32: Recess
   33: Smaller-diameter rod portion
   34: Larger-diameter rod portion
   35: Rod shoulder
   36, 37: Groove
   38: Gap
   39: Nozzle chamber
   40: Vortex-flow generator
50: Medicament having ejected from nozzle
   51: Narrower-diffusion region
   52: Wider-diffusion region
100: Upper exhaust airless-type spray/spout device
   110: Protective cap
   120: Pump
      121: Upper annular outer-peripheral surface part
      122: Lower annular outer-peripheral surface part
   130: Annular operating portion (finger rest)
   140: Medicament container
      141: Inner peripheral surface
   150: Suction chamber
   160: Sliding bottom-lid body
   170: Stationary bottom lid
   180: Vent hole
   190: Ring
   200: Syringe-type spray/spout device
      210: Medicament
      220: Syringe barrel
      230: Syringe body
      240: Plunger rod
         240a: Fixing portion
      250: Tip opening
      260: Piston
      270: Finger rest
      280: Plunger operating portion
   300: Nasal rest article for nasal administration
   350: Cylindrical part/portion

## Claims

1. A nasal spray/spout nozzle (10) for nasal administration, comprising:
a tip portion (11) having a nozzle exit port (11A); and
a nose rest (12) capable of abutting on a nose region around an external naris
wherein, in a top plan view of the nasal spray/spout nozzle (10), an axis of the tip portion (11) and a center of the nose rest (12) have an eccentric relationship with each other such that they are offset from each other;
**characterized in that**:
in the top plan view of the nasal spray/spout nozzle (10), an outer contour (11P) of the tip portion (11) has a non-surround part (11P2) where a part of the outer contour (11P) of the tip portion (11) is not surrounded by an outer contour (12P) of the nose rest (12); and contour parts situated at both sides of the non-surround part (11P2) in the outer contour (12P) of the nose rest (12) are in a form of arc.

2. The nasal spray/spout nozzle according to claim 1, wherein, in a top plan view of the nasal spray/spout nozzle (10), an outer contour (12P) of the nose rest (12) surrounds at least a part of an outer contour (11P) of the tip portion (11).

3. The nasal spray/spout nozzle according to claim 1 or 2, wherein, in a top plan view of the nasal spray/spout nozzle (10), the nose rest (12) has approximately an elliptic shape.

4. The nasal spray/spout nozzle according to any one of claims 1 to 3, wherein the nose rest (12) has a reducing portion that reduces a diameter of the nose rest (12) toward the nozzle exit port (11A).

5. The nasal spray/spout nozzle according to claim 4, wherein the nose rest (12) includes a relatively steep face and a relatively non-steep face.

6. The nasal spray/spout nozzle according to any one of claims 1 to 5, wherein an ejection direction of the nozzle exit port (11A) forms an angle with respect to an axis of the nasal spray/spout nozzle (10).

7. The nasal spray/spout nozzle according to any one of claims 1 to 6, wherein the nose rest (12) has a fit surface for fitting the nose region around the external naris.

8. The nasal spray/spout nozzle according to any one of claims 1 to 7, wherein, in a lateral plan view of the nasal spray/spout nozzle (10), a length dimension of the tip portion (11) is 10 mm or more and 25 mm or less.

9. The nasal spray/spout nozzle according to any one of claims 1 to 8, wherein the tip portion (11) has a taper part that is gradually tapered toward the nozzle exit port (11A).

10. The nasal spray/spout nozzle according to any one of claims 1 to 9, wherein an end face of the tip portion (11) has a diameter of 3 mm or more and 10 mm or less, the end face being provided with the nozzle exit port (11A).

11. The nasal spray/spout nozzle according to any one of claims 1 to 10, further comprising a finger rest (14) for putting a finger thereon.

12. The nasal spray/spout nozzle according to claim 11, in a top plan view of the nasal spray/spout nozzle (10), a longest-diameter direction of the nose rest (12) and a longest-diameter direction of the finger rest (14) are approximately perpendicular to each other.

13. An upper exhaust airless-type spray/spout device, comprising the nasal spray/spout nozzle (10) according to any one of claims 1 to 12.

14. A syringe-type spray/spout device (200), comprising the nasal spray/spout nozzle (10) according to any one of claims 1 to 10.

15. The syringe-type spray/spout device according to claim 14, wherein a syringe body (230) to which the nasal spray/spout nozzle (10) is attached comprises a finger rest (270).

16. The syringe-type spray/spout device according to claim 15, wherein, in a top plan view of the syringe-type spray/spout device (10), a longest-diameter direction of the nose rest (12) and a longest-diameter direction of the finger rest (270) are approximately perpendicular to each other.

## Patentansprüche

1. Nasenspray-/Auslassdüse (10) zur Verabreichung in die Nase, umfassend:
einen Spitzenabschnitt (11), der eine Düsenaustrittsöffnung (11A) aufweist; und
eine Nasenauflage (12), die an einem Nasenbereich um ein äußeres Nasenloch herum anliegen kann
wobei in einer Draufsicht auf die Nasenspray-/Auslassdüse (10) eine Achse des Spitzenabschnitts (11) und ein Mittelpunkt der Nasenauflage (12) eine exzentrische Beziehung zueinander aufweisen, sodass sie voneinander versetzt sind;
**dadurch gekennzeichnet, dass**:
in der Draufsicht auf die Nasenspray-/Auslassdüse (10) eine äußere Kontur (11P) des Spitzenabschnitts (11) einen nicht umschlossenen Teil (11P2) aufweist, wo ein Teil der äußeren Kontur (11P) des Spitzenabschnitts (11) nicht von einer äußeren Kontur (12P) der Nasenauflage (12) umschlossen ist; und Konturteile, die an beiden Seiten des nicht umschlossenen Teils (11P2) in der äußeren Kontur (12P) der Nasenauflage (12) gelegen sind, in einer Bogenform sind.

2. Nasenspray-/Auslassdüse nach Anspruch 1, wobei in einer Draufsicht auf die Nasenspray-/Auslassdüse (10) eine äußere Kontur (12P) der Nasenauflage (12) mindestens einen Teil einer äußeren Kontur (11P) des Spitzenabschnitts (11) umschließt.

3. Nasenspray-/Auslassdüse nach Anspruch 1 oder 2, wobei in einer Draufsicht auf die Nasenspray-/Auslassdüse (10) die Nasenauflage (12) ungefähr eine elliptische Form aufweist.

4. Nasenspray-/Auslassdüse nach einem der Ansprüche 1 bis 3, wobei die Nasenauflage (12) einen Verringerungsabschnitt aufweist, der einen Durchmesser der Nasenauflage (12) in Richtung der Düsenaustrittsöffnung (11A) verringert.

5. Nasenspray-/Auslassdüse nach Anspruch 4, wobei die Nasenauflage (12) eine relativ steile Fläche und eine relativ nicht steile Fläche einschließt.

6. Nasenspray-/Auslassdüse nach einem der Ansprüche 1 bis 5, wobei eine Ausstoßrichtung der Düsenaustrittsöffnung (11A) einen Winkel in Bezug auf eine Achse der Nasenspray-/Auslassdüse (10) bildet.

7. Nasenspray-/Auslassdüse nach einem der Ansprüche 1 bis 6, wobei die Nasenauflage (12) eine Passfläche zum Anpassen an den Nasenbereich um das äußere Nasenloch herum aufweist.

8. Nasenspray-/Auslassdüse nach einem der Ansprüche 1 bis 7, wobei in einer seitlichen Draufsicht auf die Nasenspray-/Auslassdüse (10) eine Längenabmessung des Spitzenabschnitts (11) 10 mm oder mehr und 25 mm oder weniger beträgt.

9. Nasenspray-/Auslassdüse nach einem der Ansprüche 1 bis 8, wobei der Spitzenabschnitt (11) einen sich verjüngenden Teil aufweist, der sich allmählich in Richtung der Düsenaustrittsöffnung (11A) verjüngt.

10. Nasenspray-/Auslassdüse nach einem der Ansprüche 1 bis 9, wobei eine Endfläche des Spitzenabschnitts (11) einen Durchmesser von 3 mm oder mehr und 10 mm oder weniger aufweist, wobei die Endfläche mit der Düsenaustrittsöffnung (11A) bereitgestellt ist.

11. Nasenspray-/Auslassdüse nach einem der Ansprüche 1 bis 10, weiter umfassend eine Fingerauflage (14) zum Auflegen eines Fingers auf dieselbe.

12. Nasenspray-/Auslassdüse nach Anspruch 11, wobei in einer Draufsicht auf die Nasenspray-/Auslassdüse (10) eine Richtung des längsten Durchmessers der Nasenauflage (12) und eine Richtung des längsten Durchmessers der Fingerauflage (14) ungefähr senkrecht zueinander sind.

13. Sich nach oben entleerende Spray-/Auslassvorrichtung, vom luftlosen Typ, umfassend die Nasenspray-/Auslassdüse (10) nach einem der Ansprüche 1 bis 12.

14. Spritzenartige Spray-/Auslassvorrichtung (200), umfassend die Nasenspray-/Auslassdüse (10) nach einem der Ansprüche 1 bis 10.

15. Spritzenartige Spray-/Auslassvorrichtung nach Anspruch 14, wobei ein Spritzenkörper (230), an dem die Nasenspray-/Auslassdüse (10) angebracht ist, eine Fingerauflage (270) umfasst.

16. Spritzenartige Spray-/Auslassvorrichtung nach Anspruch 15, wobei in einer Draufsicht auf die spritzenartige Spray-/Auslassvorrichtung (10) eine Richtung des längsten Durchmessers der Nasenauflage (12) und eine Richtung des längsten Durchmessers der Fingerauflage (270) ungefähr senkrecht zueinander sind.

## Revendications

1. Buse de sortie en jet/pulvérisation nasale (10) pour administration nasale, comprenant :
une portion d'embout (11) présentant un orifice (11A) de sortie de buse ; et
un appui-nez (12) apte à venir en butée sur une région de nez autour d'une narine externe,
dans laquelle un axe de la portion d'embout (11) et un centre de l'appui-nez (12) présentant, dans une vue de dessus en plan de la buse de sortie en jet/pulvérisation nasale (10), une relation excentrique l'un par rapport à l'autre de telle sorte qu'ils soient décalés l'un par rapport à l'autre ;
**caractérisée en ce que** :
dans la vue de dessus en plan de la buse de sortie en jet/pulvérisation nasale (10), un contour extérieur (11P) de la portion d'embout (11) présente une partie non périphérique (11P2) où une partie du contour extérieur (11P) de la portion d'embout (11) n'est pas entourée par un contour extérieur (12P) de l'appui-nez (12) ; et des parties de contour situées au niveau des deux côtés de la partie non périphérique (11P2) dans le contour extérieur (12P) de l'appui-nez (12) sont en forme d'arc.

2. Buse de sortie en jet/pulvérisation nasale selon la revendication 1, dans laquelle, dans une vue de dessus en plan de la buse de sortie en jet/pulvérisation nasale (10), un contour extérieur (12P) de l'appui-nez (12) entoure au moins une partie d'un contour extérieur (11P) de la portion d'embout (11).

3. Buse de sortie en jet/pulvérisation nasale selon la revendication 1 ou la revendication 2, dans laquelle, dans une vue de dessus en plan de la buse de sortie en jet/pulvérisation nasale (10), l'appui-nez (12) présente approximativement une forme elliptique.

4. Buse de sortie en jet/pulvérisation nasale selon l'une quelconque des revendications 1 à 3, dans laquelle l'appui-nez (12) présente une portion de réduction qui réduit le diamètre de l'appui-nez (12) vers l'orifice (11A) de sortie de buse.

5. Buse de sortie en jet/pulvérisation nasale selon la revendication 4, dans laquelle l'appui-nez (12) inclut une face relativement raide et une face relativement non-raide.

6. Buse de sortie en jet/pulvérisation nasale selon l'une quelconque des revendications 1 à 5, dans laquelle une direction d'éjection de l'orifice (11A) de sortie de buse forme un angle par rapport à un axe de la buse de sortie en jet/pulvérisation nasale (10).

7. Buse de sortie en jet/pulvérisation nasale selon l'une quelconque des revendications 1 à 6, dans laquelle l'appui-nez (12) présente une surface d'ajustement destinée à ajuster la région de nez autour de la narine externe.

8. Buse de sortie en jet/pulvérisation nasale selon l'une quelconque des revendications 1 à 7, dans laquelle, dans une vue latérale en plan de la buse de sortie en jet/pulvérisation nasale (10), une dimension en longueur de la portion d'embout (11) est supérieure ou égale à 10 mm et inférieure ou égale à 25 mm.

9. Buse de sortie en jet/pulvérisation nasale selon l'une quelconque des revendications 1 à 8, dans laquelle la portion d'embout (11) présente une partie effilée qui est progressivement effilée vers l'orifice (11A) de sortie de buse.

10. Buse de sortie en jet/pulvérisation nasale selon l'une quelconque des revendications 1 à 9, dans laquelle une face d'extrémité de la portion d'embout (11) présente un diamètre supérieur ou égal à 3 mm et inférieur ou égal à 10 mm, la face d'extrémité étant munie de l'orifice (11A) de sortie de buse.

11. Buse de sortie en jet/pulvérisation nasale selon l'une quelconque des revendications 1 à 10, comprenant en outre un appui-doigt (14) pour y placer un doigt.

12. Buse de sortie en jet/pulvérisation nasale selon la revendication 11, dans une vue de dessus en plan de la buse de sortie en jet/pulvérisation nasale (10), une direction en diamètre le plus long de l'appui-nez (12) et une direction en diamètre le plus long de l'appui-doigt (14) sont approximativement perpendiculaires l'une à l'autre.

13. Dispositif de sortie en jet/pulvérisation de type sans air à évacuation supérieure, comprenant la buse de sortie en jet/pulvérisation nasale (10) selon l'une quelconque des revendications 1 à 12.

14. Dispositif de sortie en jet/pulvérisation de type seringue (200), comprenant la buse de sortie en jet/pulvérisation nasale (10) selon l'une quelconque des revendications 1 à 10.

15. Dispositif de sortie en jet/pulvérisation de type seringue selon la revendication 14, dans lequel un corps de seringue (230) auquel est fixée la buse de sortie en jet/pulvérisation nasale (10) comprend un appui-doigt (270).

16. Dispositif de sortie en jet/pulvérisation de type seringue selon la revendication 15, dans lequel, dans une vue de dessus en plan du dispositif de sortie en jet/pulvérisation de type seringue (10), une direction en diamètre le plus long de l'appui-nez (12) et une direction en diamètre le plus long de l'appui-doigt (270) sont approximativement perpendiculaires l'une à l'autre.
